(19) Europäisches Patentamt
European Patent Office
Office européen des brevets

(11) EP 4 721 742 A2

(12) EUROPEAN PATENT APPLICATION

(43) Date of publication:
**08.04.2026 Bulletin 2026/15**

(21) Application number: **26153325.1**

(22) Date of filing: **16.12.2022**

(51) International Patent Classification (IPC):
***A61K 31/506*** *(2006.01)*

(52) Cooperative Patent Classification (CPC):
**C07D 401/14; A61P 9/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.12.2021 EP 21215583**

(62) Document number(s) of the earlier application(s) in accordance with Art. 76 EPC:
**25155042.2 / 4 582 086**
**22840068.5 / 4 448 511**

(71) Applicant: **Idorsia Pharmaceuticals Ltd**
**4123 Allschwil (CH)**

(72) Inventors:
- **AIGLSTORFER-HAAG, Iris**
**4416 Bubendorf (CH)**
- **ISARNO, Thomas**
**4416 Bubendorf (CH)**

(74) Representative: **Kamdzhilov, Yavor**
**Idorsia Pharmaceuticals Ltd**
**Hegenheimermattweg 91**
**4123 Allschwil (CH)**

Remarks:
This application was filed on 21-01-2026 as a divisional application to the application mentioned under INID code 62.

# (54) CLAZOSENTAN DISODIUM SALT, ITS PREPARATION AND PHARMACEUTICAL COMPOSITIONS COMPRISING THE SAME

(57) The present invention relates to clazosentan disodium salt and a process for the preparation thereof.

EP 4 721 742 A2

## Description

The present invention relates to a novel process for the preparation of clazosentan disodium salt, process intermediates, and the final product. Furthermore, the present invention relates to pharmaceutical compositions comprising clazosentan and their use as endothelin receptor antagonist in the prevention and/or treatment of diseases or disorders, where endothelin receptors are involved, notably for use in the prevention and/or treatment of cerebral vasospasm (VSP) and/or its subsequent ischemic effects/symptoms after aneurysmal subarachnoid hemorrhage (aSAH).

aSAH is a sudden life-threatening bleeding in the subarachnoid space between the brain and skull caused by the rupture of an aneurysm. In order to stop the hemorrhage, emergency surgical repair with endovascular coiling or microsurgical clipping of the aneurysm is often necessary. Mortality rates following aSAH are high (approx. 25%) with a substantial portion of the survivors experiencing neurological deficits. VSP, a strong contraction of the arteries surrounding the hemorrhage that reduces blood flow and oxygen to nearby cerebral areas, may occur between 4 and 14 days after aneurysm securing. Complications such as delayed cerebral ischemia and/or cerebral infarction in which brain tissue death occurs due to an inadequate blood supply develop after VSP.

Clazosentan is a potent endothelin ($ET_A$-selective) receptor antagonist that has been evaluated with regard to VSP and/or its subsequent ischemic effects/symptoms after aSAH in several clinical trials (e.g., NCT00111085, NCT00558311, NCT00940095, NCT02560532, NCT03585270, JapicCTI-163369, and JapicCTI-163368). In 2022 clazosentan (trade name Pivlaz) was approved in Japan (Lee A. Clazosentan: First Approval. Drugs. 2022 Apr;82(6):697-702. Doi: 10.1007/s40265-022-01708-0. PMID: 35362854).

Clazosentan disodium salt (hereinafter also referred to as the compound of Formula 6) is known under several systematic names such as 5-methyl-pyridine-2-sulfonic acid N-{6-(2-hydroxy-ethoxy)-5-(2-methoxy-phenoxy)-2-[2-(1H-tetrazol-5-yl)-pyridin-4-yl]-pyrimidin-4-yl}-amide disodium salt or 5-methyl-pyridine-2-sulfonic acid N-{6-(2-hydroxy-ethoxy)-5-(2-methoxy-phenoxy)-2-[2-(1H-tetrazol-5-yl)-pyridin-4-yl]-pyrimidin-4-yl}-amide sodium salt (1:2). Clazosentan is also known in the art under its laboratory codes e.g., ACT-108475 (free acid), AXV-034343 (free acid), AXV-034343A (disodium salt), ACT-108475A (disodium salt), VML 588, and Ro 61-1790; and may be represented by the following structure

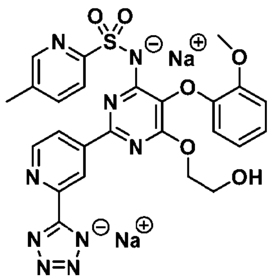

Formula 6.

EP0979822 discloses clazosentan disodium salt and a preparation thereof (Example 9). WO9619459 discloses a laboratory scale preparation of clazosentan free acid (Example 29). Clazosentan disodium salt appears to be mentioned in Example C b) of WO9619459, however, the document is silent with regard to a preparation method. EP0897914 relates to a method for the preparation of 2,5-disubstituted pyridines that may be used in the preparation of clazosentan free acid (e.g. [0018], [0024] and [0032]). EP0897914 is primarily directed to the initial manufacturing steps of a process for making clazosentan. WO2000042035 and WO2000052007 relate to heterocyclic sulfonamides as inhibitors of endothelin receptors. Vorbrüggen et al.: Synthesis 1983(4):316-319, DOI: 10.1055/s-1983-30321; T. Sakamoto, et al.:Chem.Pharm.Bull. 33, 565-571 (1985); and A. Kubo, et al.:Chem.Pharm.Bull. 36, 4355-4363 (1988) discuss cyanation of heterocyclic-N-oxides.

In accordance with the present invention novel processes suitable for large scale manufacturing of clazosentan and its disodium salt have been found. The processes may provide advantageous process characteristics/conditions such as particularly suitable reagents/solvents; high yields; high degrees of conversion; short reaction times; low temperatures and pressures; high safety and robustness; and/or good filterability of intermediates / final product. Further, the process may provide clazosentan disodium salt with advantageous purity, impurity profile, and/or correct sodium content. Also, the process may provide advantageous crystalline forms of process intermediates and/or clazosentan disodium salt. The use of said advantageous crystalline forms may simplify and reduce cost of material handling/transportation/storage/testing during manufacturing; reduce mass variation and thereby simplify dosing in drug product manufacturing; reduce agglomerate formation under storage conditions; and/or improve homogeneity of bulk material. It has been found that

the process of the current invention may provide discolored clazosentan disodium salt.

**Description of the Figures**

Figure 1 shows the X-ray powder diffractogram of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide disodium salt in crystalline form A, wherein the X-ray powder diffractogram is displayed as a function of the diffraction angle 2θ of Cu Kα radiation. The X-ray diffractogram of Figure 1 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 10% are reported): 9.4° (100%), 12.0° (19%), 13.2° (15%), 17.7° (16%), 18.4° (20%), 19.8° (18%), 21.2° (23%), 21.9° (35%), and 24.9° (15%).

Figure 2 shows the X-ray powder diffractogram of 5-methyl-pyridine-2-sulfonic acid 6-chloro-5-(2-methoxy-phenoxy)-2-(1-oxy-pyridin-4-yl)-pyrimidin-4-yl amide (free acid), wherein the X-ray powder diffractogram is displayed as a function of the diffraction angle 2θ of Cu Kα radiation. The X-ray diffractogram of Figure 2 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 7% are reported): 8.3° (100%), 9.1° (34%), 12.3° (10%), 16.7° (11%), 18.1° (10%), 20.2° (26%), 20.5° (11%), 25.0° (27%), 25.6° (7%), and 27.1° (18%).

Figure 3 shows the X-ray powder diffractogram of 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide sodium salt, wherein the X-ray powder diffractogram is displayed as a function of the diffraction angle 2θ of Cu Kα radiation. The X-ray diffractogram of

Figure 3 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 4 % are reported): 9.8° (100%), 10.4° (11%), 18.2° (21%), 18.8° (7%), 19.2° (4%), 20.9° (10%), 23.1° (17%), 26.2° (11%), 27.1° (10%), and 29.0° (12%).

Figure 4 shows the X-ray powder diffractogram of 5-methyl-pyridine-2-sulfonic acid 2-(2-cyano-pyridin-4-yl)-6-(2-hydroxy-ethoxy)-5-(2-methoxy-phenoxy)-pyrimidin-4-yl amide, wherein the X-ray powder diffractogram is displayed as a function of the diffraction angle 2θ of Cu Kα radiation. The X-ray diffractogram of Figure 4 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 10 % are reported): 7.0° (100%), 8.6° (38%), 11.6° (17%), 12.6° (25%), 13.8° (54%), 18.2° (34%), 21.4° (63%), 23.1° (30%), 25.3° (26%), and 26.7° (42%).

Figure 5 shows the X-ray powder diffractogram of, wherein the X-ray powder diffractogram of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide is displayed as a function of the diffraction angle 2θ of Cu Kα radiation. The X-ray diffractogram of Figure 5 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 20 % are reported): 6.0° (100%), 9.0° (34%), 12.7° (29%), 17.7° (35%), 18.0° (48%), 20.7° (36%), 23.0° (31%), 24.6° (66%), 25.8° (54%), and 27.2° (31%).

Figure 6 shows the X-ray powder diffractogram of, wherein the X-ray powder diffractogram of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide tetrahydrofuran solvate is displayed as a function of the diffraction angle 2θ of Cu Kα radiation. The X-ray diffractogram of Figure 6 shows peaks having a relative intensity, as compared to the most intense peak in the diffractogram, of the following percentages (relative peak intensities given in parenthesis) at the indicated angles of refraction 2theta (selected peaks from the range 5-35° 2theta with relative intensity larger or equal than 5 % are reported): 9.0° (100%), 15.4° (11%), 17.2° (6%), 19.0° (14%), 21.7° (10%), 23.6° (9%), 24.2° (19%), 24.4° (12%), 25.8° (10%), and 27.9° (15%).

For avoidance of doubt, the above-listed peaks describe the experimental results of the X-ray powder diffractograms shown in Figures 1-6. It is understood that, in contrast to the above peak lists, only a selection of characteristic peaks is required to characterize fully and unambiguously the corresponding compound in the crystalline forms of the present invention.

## Detailed Description of the Invention

1) One aspect of the present invention relates to a process comprising

(a) reacting the compound of Formula 1

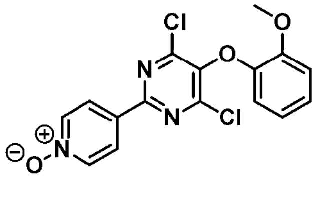

Formula 1

with 5-methyl-pyridine-2-sulfonamide or a salt (notably alkali metal salt; especially potassium salt) thereof, under basic conditions, to give the compound of Formula 2 or a salt (notably alkali metal salt; especially potassium salt) thereof

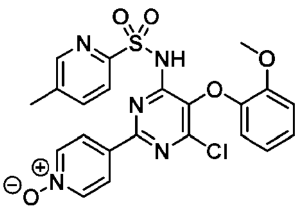

Formula 2.

The term "basic conditions" as used herein refers to the presence of compounds which are able to deprotonate the sulfonamide group in the 5-methyl-pyridine-2-sulfonamide such as basic alkali metal salts, alkali metal hydroxides, tri-$C_{1-4}$-alkyl amines, 1,4-diazabicyclo[2.2.2]octane (DABCO), amidine bases such as 1,8-diazabicyclo[5.4.0]undec-7-en (DBU) or 1,5-diazabicyclo(4.3.0)non-5-ene (DBN), pyridine which is unsubstituted, mono-, di-, or tri-substituted with $C_{1-4}$-alkyl (especially methyl). Preferred are basic alkali metal salts, alkali metal hydroxides, and tri-$C_{1-4}$-alkyl amines. Especially preferred are basic alkali metal salts or alkali metal hydroxides. The term "tri-$C_{1-4}$-alkyl amines" as used herein refers to compounds of the formula N**R¹R²R³**, wherein **R¹**, **R²**, and **R³** independently represent $C_{1-4}$-alkyl. The term $C_{1-4}$-alkyl refers to a saturated, branched or straight, hydrocarbon, monovalent group with one to four carbon atoms. Examples of $C_{1-4}$-alkyl groups are methyl, ethyl, n-propyl, isopropyl, n-butyl, tert-butyl, sec-butyl and isobutyl; especially ethyl.

Basic alkali metal salts are salts of alkali metals that hydrolyze in water to form a solution with a pH value above 7. Examples of such salts are carbonates, bicarbonates, sulfides, cyanides, or acetates, of alkali metals. Notably carbonates or bicarbonates of lithium, sodium, or potassium. Suitable alkali metal hydroxides are hydroxides of sodium, potassium, lithium, or cesium; especially sodium hydroxide, potassium hydroxide, and lithium hydroxide.

2) Another embodiment relates to the process according to embodiment 1), wherein step (a) is performed in the presence of a basic alkali metal salt (especially potassium carbonate).

3) Another embodiment relates to the process according to embodiment 2), wherein the basic alkali metal salt (especially potassium carbonate) is from about 1 equivalent to about 3 equivalents (in particular about 2 equivalents) [with respect to the amount of the compound of Formula 1].

4) Another embodiment relates to the process according to any one of embodiments 1) to 3), wherein step (a) is performed in a polar aprotic organic solvent or a mixture of such solvents (notably acetonitrile, 1,4-dioxane, or a mixture thereof; especially acetonitrile).

It is understood that the term "polar aprotic organic solvent" in the context of the present invention is an organic

solvent which has a dielectric constant "ε" larger than 15, especially larger than 20 (measured at 25°C), and lacks an acidic hydrogen atom. For example, such solvent may be acetone, ethyl acetate, dimethyl sulfoxide, 1,4-dioxane, acetonitrile, N-methyl-pyrrolidone, or dimethyl-formamide.

5) Another embodiment relates to the process according to any one of embodiments 1) to 4), wherein 5-methylpyridine-2-sulfonamide is in an amount from about 1 equivalent to about 1.5 equivalents (especially about 1 equivalent) [with respect to the amount of the compound of Formula 1].

6) Another embodiment relates to the process according to any one of embodiments 1) to 5), wherein the compound of Formula 2 is isolated in the form of a

- potassium salt i.e. the compound of Formula 2a (especially a hydrate thereof)

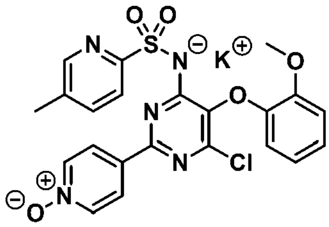

Formula 2a;

- or especially in the form of a free acid.

The process according to any one of embodiments 1) to 6) may be used for the preparation of the compound of Formula 6.

7) Another aspect of the present invention relates to a process comprising

(b) reacting the compound of Formula 2 or a salt (notably alkali metal salt; especially potassium salt) thereof

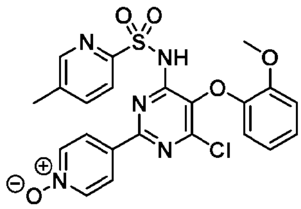

Formula 2

with ethylene glycol in the presence of an alkali metal hydroxide (especially sodium hydroxide), to give the compound of Formula 3 or a salt (notably sodium salt; especially sodium salt hydrate) thereof

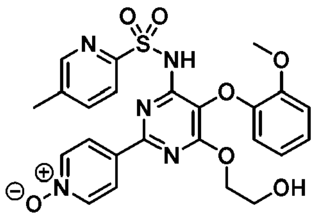

Formula 3.

8) Another embodiment relates to the process according to embodiment 7), wherein the compound of Formula 2 is reacted (i.e. compound of Formula 2 in free acid form).

9) Another embodiment relates to the process according to any one of embodiments 7) or 8), wherein the alkali metal hydroxide (especially sodium hydroxide) is in an amount of at least about 4 equivalents (notably at least about 5 equivalents; especially about 5.7 equivalents) [with respect to the amount of the compound of Formula 2].

10) Another embodiment relates to the process according to any one of embodiments 7) to 9), wherein step (b) is performed in the presence of at least one free radical scavenger (especially 2,6-di-tert-butyl-4-methylphenol). Notably, the free radical scavenger is present in an amount from about 0.1 to about 1 equivalents (especially about 0.5 equivalents)

[with respect to the compound of Formula 2 in free acid form].

The term "free radical scavenger" in the context of the present invention refers to a compound which reacts with free radical species formed during the process and thereby prevents the formation of by-products. Examples of such compounds are 2,6-di-tert-butyl-4-methylphenol (BHT), 2-(1,1-dimethylethyl)-1,4-benzenediol, 1,2-dihydro-6-ethoxy-2,2,4-trimethylquinoline, tertiary butylhydroquinone (TBHQ), propyl gallate, dodecyl gallate, gallic acid, or butylated hydroxyanisole such as the isomeric mixture of 2-tert-butyl-4-hydroxyanisole and 3-tert-butyl-4-hydroxyanisole (BHA); especially 2,6-di-tert-butyl-4-methylphenol (BHT). When a free radical scavenger such as 6-di-tert-butyl-4-methylphenol is used, toluene may be added to the reaction mixture.

11) Another embodiment relates to the process according to any one of embodiments 7) to 10), wherein the compound of Formula 3 is isolated in form of a sodium salt (especially a hydrate thereof), i.e. the compound of Formula 3a (especially a hydrate thereof)

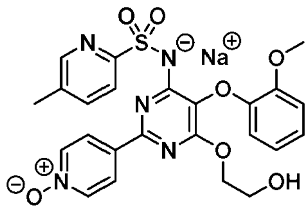

Formula 3a.

The process according to any one of embodiments 7) to 11) may be used for the preparation of the compound of Formula 6.

12) Another aspect of the present invention relates to a process comprising

(c) reacting the compound of Formula 3 or a salt (notably sodium salt; especially sodium salt hydrate) thereof

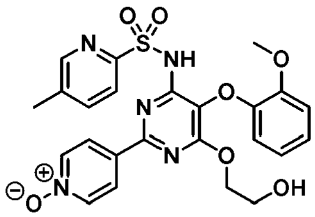

Formula 3

with trimethylsilyl cyanide in the presence of a mono-, di-, or tri-$C_{1-4}$-alkyl amine (especially triethylamine), to give the compound of Formula 4

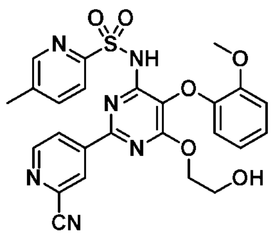

Formula 4.

13) Another embodiment relates to the process according to embodiment 12), wherein the compound of Formula 3 is reacted in form of a salt, especially in form of the sodium salt (Formula 3a) thereof.

14) Another embodiment relates to the process according to any one of embodiments 12) or 13), wherein step (c) is performed in the presence of trimethylsilyl chloride. Notably, wherein the trimethylsilyl chloride is in an amount from about 1 to about 2 equivalents [with respect to the compound of Formula 3/3a] (especially about 1.3 equivalents).

It is understood that the amount of trimethylsilyl chloride may further be increased when additional alcohol moieties such as residual ethylene glycol are present in the starting materials.

15) Another embodiment relates to the process according to any one of embodiments 12) to 14), wherein in the

trimethylsilyl cyanide is in an amount from about 3 to about 6 (especially about 4.5 equivalents) [with respect to the compound of Formula 3/3a].

16) Another embodiment relates to the process according to any one of embodiments 12) to 15), wherein mono-, di-, or tri-$C_{1-4}$-alkyl amine is in an amount from about 2 to about 4 (especially 3.1 equivalents) [with respect to the compound of Formula 3/3a].

17) Another embodiment relates to the process according to any one of embodiments 12) to 16), wherein step (c) is performed under pressure higher than atmospheric pressure (i.e. higher than about 1 bar; notably from about 2 to about 10 bar; especially at about 6 bar).

18) Another embodiment relates to the process according to any one of embodiments 12) to 17), wherein step (c) is performed at a temperature from about 70°C to about 110°C (notably from about 80°C to about 100°C especially at about 93°C).

The process according to any one of embodiments 12) to 18) may be used for the preparation of the compound of Formula 6.

19) Another aspect of the present invention relates to a process comprising

(d) reacting the compound of Formula 4

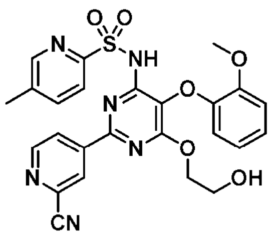

Formula 4

with an alkali metal azide (especially sodium azide) in the presence of ammonium chloride, to give the compound of Formula 5 or a solvate (especially tetrahydrofuran solvate) thereof;

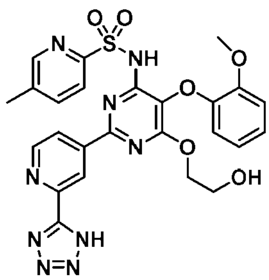

Formula 5.

20) Another embodiment relates to a process according to embodiment 19), wherein the compound of Formula 5 is isolated (by solid-liquid separation) adsorbed on a filter aid, said filter aid comprising kieselguhr, activated carbon, or perlite; especially kieselguhr.

It is understood that isolating the solid residue of the compound of Formula 5 refers to separating the solid phase of a suspension from its liquid phase. It is understood that said isolation may be performed by any method for solid-liquid separation such as filtration (e.g. gravity filtration, or notably vacuum filtration).

The term "filter aid" as used herein refers to a carrier consisting of solid particles that improves flow rate and/or filtrate clarity by e.g. decreasing cake compressibility and/or improving cake permeability/porosity. Filter aids comprise porous particles and are either added to the suspension to be filtered or placed on the filter as a layer through which the suspension to be filtered must pass. Examples of filter aids are kieselguhr (also known as kieselgur, diatomaceous earth, or diatomite) such as Hyflo® Super Cel® NF, activated carbon, perlite, (wood)cellulose, agricultural fibers, saw dust, (calcinated) rice hull ash, or paper fibers (e.g. fibers from used newspapers); especially suitable filter aids are kieselguhr, activated carbon, or perlite; in particular kieselguhr.

21) Another embodiment relates to a process according to embodiment 20), wherein the amount of the filter aid used is at least about 10% w/w (notably at least about 50% w/w; especially at least 75% w/w; in particular at least 100%) of the amount of the compound of Formula 4.

22) Another embodiment relates to a process according to any one of embodiments 19) to 21), wherein step (d) is

performed at a temperature of less than about 110°C (notably from about 80°C to about 110°C; especially at about 95°C).

23) Another embodiment relates to a process according to any one of embodiments 19) to 22), wherein step (d) is performed in dimethylformamide.

24) Another embodiment relates to a process according to any one of embodiments 19) to 23), wherein the adsorbed compound of Formula 5 is desorbed by dissolution in an appropriate solvent [e.g. in tetrahydrofuran (notably at a temperature of greater than about 50°C, especially at about 66°C)], and the compound of Formula 5 is isolated as a solvate e.g. tetrahydrofuran solvate (notably by precipitating and/or crystallization from appropriate solvent e.g. tetrahydrofuran; e.g. at a temperature of below about 20°C, especially at about 10°C; wherein said compound is especially isolated through a solid-liquid separation and optionally further slurried in an appropriate solvent (e.g. tetrahydrofuran), especially under reflux).

The process according to any one of embodiments 19) to 24) may be used for the preparation of the compound of Formula 6.

25) Another aspect relates to a process comprising the following steps

(e) reacting the compound of Formula 5 or a solvate (notably tetrahydrofuran solvate; especially tetrahydrofuran mono-solvate) thereof

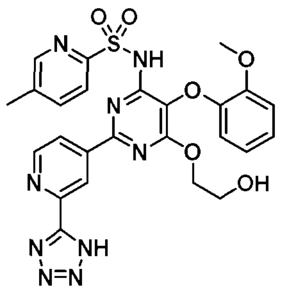

Formula 5

with a sodium containing base (notably sodium alkoxide, sodium hydride, or sodium hydroxide; especially sodium alkoxide containing from one to four carbon atoms; in particular sodium methoxide), to give the compound of Formula 6

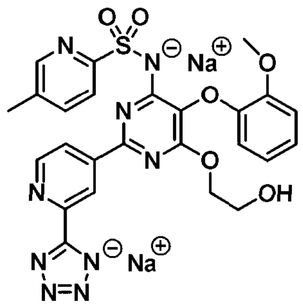

Formula 6;

(f) optionally purifying the compound of Formula 6 by

(f1) recrystallizing the compound of Formula 6 [of step (e)] in water at pH ≥ 7 (notably pH ≥ 10; especially pH of about 14); and/or

(f2) triturating the compound of Formula 6 [of step (e) or step (f1)] in a solvent selected from a lower alcohol or a mixture of lower alcohols; and

(g) optionally drying the compound of Formula 6.

In a preferred sub-embodiment of embodiment 25), step (f) and step (g) are comprised in the process according to embodiment 25) (i.e. step (f) and step (g) are not optional). In a further preferred sub-embodiment of embodiment 25), step (f) and step (g) are comprised in the process according to embodiment 25) (i.e. step (f) and step (g) are not optional), wherein step (f) comprises step (f1), wherein step (f2) is absent. Yet in a further preferred sub-embodiment, step (f) and step (g) are comprised in the process according to embodiment 25) (i.e. step (f) and step (g) are not optional), wherein step

(f) comprises step (f1) and step (f2). Yet in another preferred sub-embodiment of embodiment 25), step (f) and step (g) are comprised in the process according to embodiment 25) (i.e. step (f) and step (g) are not optional), wherein step (f) comprises step (f2), wherein step (f1) is absent.

The term "sodium containing base" as used herein refers to an organic and/or inorganic Brønsted base comprising sodium; notably, wherein said base is suitable for accepting protons ($H^+$ ions) from the compound of Formula 5 such that the compound of Formula 6 is produced. Suitable Brønsted bases may be sodium alkoxides (e.g. sodium methoxide, sodium ethoxide, sodium n-propoxide, sodium iso-propoxide, and/or sodium butoxide), sodium hydride, sodium oxide, and/or sodium hydroxide; notably sodium alkoxide, sodium hydride, or sodium hydroxide; especially sodium alkoxide containing from one to four carbon atoms; in particular sodium methoxide. The term "recrystallizing" as used herein refers to dissolving (the solid residue of) the compound of Formula 6 obtained in step (e) in water at pH ≥ 7 (notably at a weight ratio of the compound of Formula 6 / water equal to about 1 to 3) at elevated temperatures (e.g. at about 60 to 80°C), cooling the solution, and isolating (the solid residue of) the compound of Formula 6 (notably by solid-liquid separation). The value of pH ≥ 7 (notably pH ≥ 10; especially pH of about 14) referred to in the recrystallization step of embodiment 25) may be achieved by the addition of a base such as alkali metal base; especially by the addition of sodium hydroxide.

The term "triturating" as used herein refers to a process used to purify crude organic chemical compounds containing soluble impurities. A solvent is chosen in which the desired product is insoluble, and the undesired by-products are soluble, or vice versa. For example, when the impurities are soluble and the desired product is not, the crude material is washed with the solvent and filtered, leaving the purified product in solid form and any impurities in solution. In particular, the term "triturating" (also referred to as slurring) as used herein refers to

- suspending (the solid residue of) the compound of Formula 6 [obtained in step (f1) or step (e)] in a solvent selected from a lower alcohol or a mixture of lower alcohols;
- optionally agitating (e.g. stirring) the suspension for a predefined amount of time, especially under heating; and
- isolating (the solid residue of) the compound of Formula 6.

It is understood that the suspending step is notably performed at temperatures above room temperature, especially at about 70°C. The agitating step is notably performed at temperatures above room temperature, especially at reflux. The isolating step is notably preceded by a cooling step to temperatures below room temperature, especially below 0°C. It is further understood that isolating (the solid residue of) the compound of Formula 6 refers to separating the solid phase of a suspension from its liquid phase. This may be performed by any method for solid-liquid separation such as filtration (e.g. centrifugal filtration, gravity filtration, or vacuum filtration), sedimentation, decantation, and/or centrifugation; especially by gravity or vacuum filtration.

It is understood that the drying of step (g) may be performed at temperatures above room temperature (especially from about 25°C to about 75°C), optionally under vacuum.

The term "lower alcohol(s)" as used hereinabove and hereinbelow refers to saturated straight or branched monovalent alkanol(s) containing from one to five (notably one to three; especially one or two) carbon atoms. Examples of lower alcohols are methanol, ethanol, 1-propanol, 2-propanol, 1-butanol, isobutanol, 2-methyl-propan-2-ol, 2-methyl-propan-1-ol, 1-pentanol, 2-pentanol, or 3-pentanol.

26) Another embodiment relates to the process according to embodiment 25), wherein the sodium containing base (notably the sodium alkoxide; in particular sodium methoxide) is from 2 to 3 (especially about 2.5) equivalents with respect to the compound of Formula 5.

27) Another embodiment relates to the process according to any one of embodiments 25) or 26), wherein step (e) is performed in a lower alcohol or mixture of lower alcohols (notably methanol, ethanol, 1-propanol, 2-propanol, or a mixture thereof; especially methanol, ethanol, or a mixture thereof; in particular a mixture of 1 wt. methanol and 3 wt. ethanol).

28) Another embodiment relates to the process according to any one of embodiments 25) to 27), wherein the solvent used for triturating in step (f2) is selected from methanol, ethanol, 1-propanol, 2-propanol, or a mixture thereof [notably methanol, ethanol, or a mixture thereof; especially a mixture of methanol and ethanol (in particular in a ratio of 1 wt. methanol and 3 wt. ethanol).

The process according to any one of embodiments 25) to 28) may be used for the preparation of the compound of Formula 6.

29) Another aspect of the present invention relates to a process for the preparation of the compound of Formula 6, said process comprising step (e), and optionally comprising steps (f) and (g), according to any one of embodiments 25) to 28); said process further comprising the steps

- step (d) according to any one of embodiments 19) to 24);
  OR

- step (d) according to any one of embodiments 19) to 24); and
  step (c) according to any one of embodiments 12) to 18) [especially step (c) according to any one of embodiments 14) to 18)];
  OR

- step (d) according to any one of embodiments 19) to 24);

  step (c) according to any one of embodiments 12) to 18) [especially step (c) according to any one of embodiments 14) to 18)]; and
  step (b) according to any one of embodiments 7) to 11) [especially step (b) according to any one of embodiments 9) to 11)];
  OR

- step (d) according to any one of embodiments 19) to 24);

  step (c) according to any one of embodiments 12) to 18) [especially step (c) according to any one of embodiments 14) to 18)];
  step (b) according to any one of embodiments 7) to 11) [especially step (b) according to any one of embodiments 9) to 11)]; and
  step (a) according to any one of embodiments 1) to 6).

30) Another aspect of the present invention relates to a process for the preparation of the compound of Formula 6, comprising step (e), (f), and (g), according to any one of embodiments 25) to 28); said process further comprising the steps:

- step (d) according to any one of embodiments 19) to 24);
  OR

- step (d) according to any one of embodiments 19) to 24); and
  step (c) according to any one of embodiments 12) to 18) [especially step (c) according to any one of embodiments 14) to 18)];
  OR

- step (d) according to any one of embodiments 19) to 24);

  step (c) according to any one of embodiments 12) to 18) [especially step (c) according to any one of embodiments 14) to 18)]; and
  step (b) according to any one of embodiments 7) to 11) [especially step (b) according to any one of embodiments 9) to 11)];
  OR

- step (d) according to any one of embodiments 19) to 24);

  step (c) according to any one of embodiments 12) to 18) [especially step (c) according to any one of embodiments 14) to 18)];
  step (b) according to any one of embodiments 7) to 11) [especially step (b) according to any one of embodiments 9) to 11)]; and
  step (a) according to any one of embodiments 1) to 6).

31) Another aspect of the present invention relates to a process for the preparation of the compound of Formula 6, comprising

- step (a) according to any one of embodiments 1) to 6);

  step (b) according to any one of embodiments 7) to 11);
  step (c) according to any one of embodiments 12) to 18) [especially step (c) according to any one of embodiments 14) to 18)];
  step (d) according to any one of embodiments 19) to 24);
  step (e) according to any one of embodiments 25) to 28);

step (f) according to any one of embodiments 25) to 28); and
step (g) according to any one of embodiments 25) to 28).

A sub-embodiment of embodiment 31) relates to a process for preparation of the compound of Formula 6, said process comprising step (a) according to any one of embodiments 1) to 6); step (b) according to embodiment 7); step (c) according to embodiment 12); step (d) according to embodiment 19); step (e) according to embodiment 25).

A sub-embodiment of embodiment 31) relates to a process for preparation of the compound of Formula 6, said process comprising step (a) according to embodiment 1); step (b) according to any one of embodiments 7) to 11); step (c) according to embodiment 12); step (d) according to embodiment 19); step (e) according to embodiment 25).

A sub-embodiment of embodiment 31) relates to a process for preparation of the compound of Formula 6, said process comprising step (a) according to embodiment 1); step (b) according to embodiment 7); step (c) according to any one of embodiments 12) to 18); step (d) according to embodiment 19); step (e) according to embodiment 25).

A sub-embodiment of embodiment 31) relates to a process for preparation of the compound of Formula 6, said process comprising step (a) according to embodiment 1); step (b) according to embodiment 7); step (c) according to embodiment 12); step (d) according to any one of embodiments 19) to 24); step (e) according to embodiment 25).

32) Another aspect of the present invention relates to a process for the preparation of the compound of Formula 6, according to embodiment 31), wherein step (f) is optional.

33) Another aspect of the present invention relates to a process for the preparation of the compound of Formula 6, according to embodiment 31), wherein step (g) is optional.

34) Another aspect of the present invention relates to a process for the preparation of the compound of Formula 6, according to embodiment 31), wherein step (f) and step (g) are optional.

It is to be understood that the individual process steps (a), (b), (c), etc. as disclosed herein may be performed in an alphabetical order, that is, process step (a) precedes process step (b); process step (b) precedes process step (c); etc. However, merely for reasons of clarity the individual process steps may be presented in a claim/embodiment of the current application in an order different than alphabetical. For example, the following order of presentation of said steps is possible and intended: process step (b), process step (a), process step (c), etc.; or process step (c), process step (a), process step (b), etc. Step (f) comprises optionally step (f1) and step (f2). If step (f1) is absent, step (f) comprises only step (f2). Step (f1), if present, precedes step (f2).

It is to be understood that the processes disclosed in the present application, especially the processes according to any one of embodiments 1) to 34), are suitable for large scale manufacturing, notably for the preparation of more than 1 kg product (intermediate or final product), notably more than 5 kg product, especially more than 10 kg product. The process steps (e) and (f) may be particularly suitable for the preparation of more than 1 kg (notably more than 3 kg; especially more than 5 kg; in particular more than 10 kg) of the compound of Formula 6.

35) Another aspect relates to the compound of Formula 2a or a hydrate thereof. A sub-embodiment relates to the compound of Formula 2a or a hydrate thereof, obtainable by step (a) of the process according to any one of embodiments 1) to 6).

35a) A further embodiment relates to a crystalline form of the compound of Formula 2; or to a crystalline form of the compound of Formula 2 obtainable by step (a) of the process according to any one of embodiments 1) to 6); characterized by the presence of peaks in the X-ray powder diffractogram at the following angles of refraction 2θ: 8.3°, 9.1°, and 25.0°.

35b) A further embodiment relates to a crystalline form of the compound of Formula 2; or to a crystalline form of the compound of Formula 2 obtainable by step (a) of the process according to any one of embodiments 1) to 6); characterized by the presence of peaks in the X-ray powder diffractogram at the following angles of refraction 2θ: 8.3°, 9.1°, 20.2°, 25.0°, and 27.1°.

35c) A further embodiment relates to a crystalline form of the compound of Formula 2; or to a crystalline form of the compound of Formula 2 obtainable by step (a) of the process according to any one of embodiments 1) to 6); characterized by the presence of peaks in the X-ray powder diffractogram at the following angles of refraction 2θ: 8.3°, 9.1°, 12.3°, 16.7°, 18.1°, 20.2°, 20.5°, 25.0°, 25.6°, and 27.1°.

35d) A further embodiment relates to a crystalline form of the compound of Formula 2; or to a crystalline form of the compound of Formula 2 obtainable by step (a) of the process according to any one of embodiments 1) to 6); characterized

- by an X-ray powder diffractogram with at least four peaks, or notably at least six peaks, or especially at least eight peaks having an angle of refraction 2θ selected from: 8.3°, 9.1°, 12.3°, 16.7°, 18.1°, 20.2°, 20.5°, 25.0°, 25.6°, and 27.1°; or
- in that an X-ray powder diffractogram essentially shows the pattern as depicted in Figure 2.

36) Another aspect relates to the compound of Formula 3a or a hydrate thereof. A sub-embodiment relates to the

compound of Formula 3a or a hydrate thereof, obtainable by step (b) of the process according to any of embodiments 7) to 11).

36a) A further embodiment relates to a crystalline form of the compound of Formula 3a (or a hydrate thereof), according to embodiment 36); or to a crystalline form of compound of Formula 3a (or a hydrate thereof), according to embodiment 36), obtainable by step (b) of the process according to any of embodiments 7) to 11); characterized by the presence of peaks in the X-ray powder diffractogram at the following angles of refraction 2θ: 9.8°, 18.2°, and 23.1°.

36b) A further embodiment relates to a crystalline form of the compound of Formula 3a (or a hydrate thereof), according to embodiment 36); or to a crystalline form of compound of Formula 3a (or a hydrate thereof), according to embodiment 36), obtainable by step (b) of the process according to any of embodiments 7) to 11); characterized by the presence of peaks in the X-ray powder diffractogram at the following angles of refraction 2θ: 9.8°, 18.2°, 23.1°, 26.2°, and 29.0°.

36c) A further embodiment relates to a crystalline form of the compound of Formula 3a (or a hydrate thereof), according to embodiment 36); or to a crystalline form of compound of Formula 3a (or a hydrate thereof), according to embodiment 36), obtainable by step (b) of the process according to any of embodiments 7) to 11); characterized by the presence of peaks in the X-ray powder diffractogram at the following angles of refraction 2θ: 9.8°, 10.4°, 18.2°, 18.8°, 19.2°, 20.9°, 23.1°, 26.2°, 27.1°, and 29.0°.

36d) A further embodiment relates to a crystalline form of the compound of Formula 3a or a hydrate thereof, according to embodiment 36); or to a crystalline form of compound of Formula 3a (or a hydrate thereof), according to embodiment 36), obtainable by step (b) of the process according to any of embodiments 7) to 11); characterized

- by an X-ray powder diffractogram with at least four peaks, or notably at least six peaks, or especially at least eight peaks having an angle of refraction 2θ selected from: 9.8°, 10.4°, 18.2°, 18.8°, 19.2°, 20.9°, 23.1°, 26.2°, 27.1°, and 29.0°; or
- in that an X-ray powder diffractogram essentially shows the pattern as depicted in Figure 3.

36e) A further embodiment relates to a crystalline form of the compound of Formula 4; or to a crystalline form of compound of Formula 4, obtainable by step (c) of the process according to any of embodiments 12) to 18); characterized by the presence of peaks in the X-ray powder diffractogram at the following angles of refraction 2θ: 7.0°, 13.8°, and 21.4°.

36f) A further embodiment relates to a crystalline form of the compound of Formula 4; or to a crystalline form of compound of Formula 4, obtainable by step (c) of the process according to any of embodiments 12) to 18); characterized by the presence of peaks in the X-ray powder diffractogram at the following angles of refraction 2θ: 7.0°, 8.6°, 13.8°, 21.4°, and 26.7°.

36g) A further embodiment relates to a crystalline form of the compound of Formula 4; or to a crystalline form of compound of Formula 4, obtainable by step (c) of the process according to any of embodiments 12) to 18); characterized by the presence of peaks in the X-ray powder diffractogram at the following angles of refraction 2θ: 7.0°, 8.6°, 11.6°, 12.6°, 13.8°, 18.2°, 21.4°, 23.1°, 25.3°, and 26.7°.

36h) A further embodiment relates to a crystalline form of the compound of Formula 4; or to a crystalline form of compound of Formula 4, obtainable by step (c) of the process according to any of embodiments 12) to 18); characterized

- by an X-ray powder diffractogram with at least four peaks, or notably at least six peaks, or especially at least eight peaks having an angle of refraction 2θ selected from: 7.0°, 8.6°, 11.6°, 12.6°, 13.8°, 18.2°, 21.4°, 23.1', 25.3°, and 26.7°; or
- in that an X-ray powder diffractogram essentially shows the pattern as depicted in Figure 4.

36i) A further embodiment relates to a crystalline form of the compound of Formula 4, especially according to any one of embodiments 36e) to 36h); or to a crystalline form of compound of Formula 4, especially according to any one of embodiments 36e) to 36h), obtainable by step (c) of the process according to any of embodiments 12) to 18); characterized by an endothermic peak in the differential scanning calorimetry thermogram (DSC2) at about 210°C (notably 210±5°C; especially at 210±2°C). [i.e. characterized by a melting point at about 210°C; notably 210±5°C; especially at 210±2°C].

For avoidance of doubt, it is understood that the crystalline form of embodiment 36i) may be characterized solely by its peak in the differential scanning calorimetry thermogram (i.e. by its melting point), or by a combination of said peak and the X-ray powder diffractogram angles of refraction specified in any one of embodiments 36e) to 36h).

37). Another aspect relates to the tetrahydrofuran solvate (especially tetrahydrofuran mono-solvate) of compound of Formula 5. A sub-embodiment relates to the tetrahydrofuran solvate (especially tetrahydrofuran mono-solvate) of compound of Formula 5, obtainable by step (d) of the process according to embodiment 19) or 24).

37a) A further embodiment relates to a crystalline form of the tetrahydrofuran solvate (especially tetrahydrofuran mono-solvate) of the compound of Formula 5 according to embodiment 37); or to a crystalline form of the tetrahydrofuran solvate (especially tetrahydrofuran mono-solvate) of the compound of Formula 5 according to embodiment 37), obtainable

by step (d) of the process according to any of embodiments 19) to 24); characterized by the presence of peaks in the X-ray powder diffractogram at the following angles of refraction 2θ: 9.0°, 24.2°, and 27.9°.

37b) A further embodiment relates to a crystalline form of the tetrahydrofuran solvate (especially tetrahydrofuran mono-solvate) of the compound of Formula 5 according to embodiment 37); or to a crystalline form of the tetrahydrofuran solvate (especially tetrahydrofuran mono-solvate) of the compound of Formula 5 according to embodiment 37), obtainable by step (d) of the process according to any of embodiments 19) to 24); characterized by the presence of peaks in the X-ray powder diffractogram at the following angles of refraction 2θ: 9.0°, 15.4°, 19.0°, 24.2°, and 27.9°.

37c) A further embodiment relates to a crystalline form of the tetrahydrofuran solvate (especially tetrahydrofuran mono-solvate) of the compound of Formula 5 according to embodiment 37); or to a crystalline form of the tetrahydrofuran solvate (especially tetrahydrofuran mono-solvate) of the compound of Formula 5 according to embodiment 37), obtainable by step (d) of the process according to any of embodiments 19) to 24); characterized by the presence of peaks in the X-ray powder diffractogram at the following angles of refraction 2θ: 9.0°, 15.4°, 17.2°, 19.0°, 21.7°, 23.6°, 24.2°, 24.4°, 25.8°, and 27.9°.

37d) A further embodiment relates to a crystalline form of the tetrahydrofuran solvate (especially tetrahydrofuran mono-solvate) of the compound of Formula 5 according to embodiment 37); or to a crystalline form of the tetrahydrofuran solvate (especially tetrahydrofuran mono-solvate) of the compound of Formula 5 according to embodiment 37), obtainable by step (d) of the process according to any of embodiments 19) to 24); characterized

- by an X-ray powder diffractogram with at least four peaks, or notably at least six peaks, or especially at least eight peaks having an angle of refraction 2θ selected from: 9.0°, 15.4°, 17.2°, 19.0°, 21.7°, 23.6°, 24.2°, 24.4°, 25.8°, and 27.9°; or
- in that an X-ray powder diffractogram essentially shows the pattern as depicted in Figure 6.

37e) A further embodiment relates to a crystalline form of the compound of Formula 5; or to a crystalline form of compound of Formula 5, obtainable by step (d) of the process according to any of embodiments 19) to 24); characterized by the presence of peaks in the X-ray powder diffractogram at the following angles of refraction 2θ: 6.0°, 24.6°, and 25.8°.

37f) A further embodiment relates to a crystalline form of the compound of Formula 5; or to a crystalline form of compound of Formula 5, obtainable by step (d) of the process according to any of embodiments 19) to 24); characterized by the presence of peaks in the X-ray powder diffractogram at the following angles of refraction 2θ: 6.0°, 9.0°, 18.0°, 24.6°, and 25.8°.

37g) A further embodiment relates to a crystalline form of the compound of Formula 5; or to a crystalline form of compound of Formula 5, obtainable by step (d) of the process according to any of embodiments 19) to 24); characterized by the presence of peaks in the X-ray powder diffractogram at the following angles of refraction 2θ: 6.0°, 9.0°, 12.7°, 17.7°, 18.0°, 20.7°, 23.0°, 24.6°, 25.8°, and 27.2°.

37h) A further embodiment relates to a crystalline form of the compound of Formula 5; or to a crystalline form of compound of Formula 5, obtainable by step (d) of the process according to any of embodiments 19) to 24); characterized

- by an X-ray powder diffractogram with at least four peaks, or notably at least six peaks, or especially at least eight peaks having an angle of refraction 2θ selected from: 6.0°, 9.0°, 12.7°, 17.7°, 18.0°, 20.7°, 23.0°, 24.6°, 25.8°, and 27.2°;or
- in that an X-ray powder diffractogram essentially shows the pattern as depicted in Figure 5.

38) Another aspect relates to the compound of Formula 5 adsorbed on a filter aid, said filter aid comprising kieselguhr, activated carbon, or perlite; especially kieselguhr. A sub-embodiment relates to the compound of Formula 5 adsorbed on a filter aid, said filter aid comprising kieselguhr, activated carbon, or perlite; especially kieselguhr; obtainable by step (d) of the process according to any one of embodiments 19) to 23).

39) Another aspect of the invention relates to the compound of Formula 6 (clazosentan disodium salt), obtainable by a process according to any one of embodiments 25) to 34).

40) Another embodiment relates to the compound of Formula 6 (clazosentan disodium salt); or to the compound according to embodiment 39); characterized in that a test solution of 2.5% w/v of said compound in water is equally or less colored than any one of reference solutions $Y_3$, $BY_3$, $GY_3$, or (optionally) $B_3$; as determined according to Chapter 2.2.2. of the European Pharmacopoeia 6.0. In a sub-embodiment of embodiment 40), the test solution is equally or less colored than reference solutions $GY_3$; as determined according to Chapter 2.2.2. of the European Pharmacopoeia 6.0.

The degree of coloration of solutions of pharmaceutical active ingredients is widely used general method which may be performed by visual color matching as described in the European Pharmacopoeia 6.0 (Ph. Eur.), Chapter 2.2.2 (01/2008:20202), Method I or II (especially Method II). In general, from three parent solutions of cobaltous (II) chloride (red), ferrous (III) chloride (yellow) and cuprous (II) sulphate (blue), and 1% hydrochloric acid, a total of 37 color reference solutions for yellow ($Y_1$-$Y_7$), greenish-yellow ($GY_1$-$GY_7$), brownish-yellow ($BY_1$-$BY_7$), brown ($B_1$-$B_9$), and red ($R_1$-$R_7$) are

prepared. The following steps of determining the degree of coloration of a solution are described in the Ph. Eur: i) A test solution of the substance under investigation (clazosentan in the present application) is prepared by dissolving a predefined amount thereof in an appropriate solvent such as water in a colorless glass vial. ii) Color reference solutions for yellow ($Y_1$ to $Y_7$), greenish-yellow ($GY_1$ to $GY_7$), brownish-yellow ($BY_1$ to $BY_7$), brown ($B_1$ to $B_9$) and red ($R_1$ to $R_7$) are either prepared according to Ph. Eur. 6.0, Chapter 2.2.2 or purchased as ready-to-use standard solutions (e.g. from Sigma-Aldrich or Merck). iii) The colors of the test solution and the reference solutions are compared visually according to Method I or Method II and the degree of coloration of the solution of the tested substance is established. Each reference solution is defined in the color space defined by the International Commission on Illumination e.g. by lightness, hue and chroma (CIELAB color space). A pertinent description of the exact methodology used in the in the present invention is found in Chapter 2.2.2 (01/2008:20202), Method I or II (preferred is Method II), of the European Pharmacopoeia 6.0. Said methodology is herein incorporated by reference. It is understood that several different editions of European Pharmacopeia may describe essentially the same method for determination of coloration such as at least European Pharmacopeia 6.0 and 7.0. In the expression "$Y_P$, $BY_Q$, $GY_R$, or $B_S$" (e.g. $Y_3$, $BY_3$, $GY_3$, or $B_3$) as used herein, the subscripts P, Q, and R independently represent integers from 1 to 7. The subscript S represents an integer from 1 to 9. For avoidance of doubt, when the coloration of a test solution is referred to as equally or less colored than any one of reference solutions $Y_P$, $BY_Q$, $GY_R$, or $B_S$, this means that the test solution is equally or less colored than a reference solution $Y_P$, or reference solution $BY_Q$, or reference solution $GY_R$, or reference solution $B_S$. This may be determined by comparison of the test solution against reference solutions $Y_1$ to $Y_7$, or against reference solutions $GY_1$ to $GY_7$, or against reference solutions $BY_1$ to $BY_7$, or against reference solutions $B_1$ to $B_9$, as appropriate. It is further understood that a test solution may be compared to only one of above-mentioned series of reference solutions (specially to reference solutions $GY_1$ to $GY_7$), wherein said series of reference solutions represents the closest color match to the color of the test solution e.g. a greenish-yellow test solution is compared to reference solutions $GY_1$ to $GY_7$; a brownish-yellow test solution is compared to reference solutions $BY_1$ to $BY_7$, etc. In the cases where color matching is possible against two or more of the above series of reference solutions, the comparison may be performed against two or more series of reference solutions.

41) Another embodiment relates to the compound of Formula 6 (clazosentan disodium salt); or to the compound according to embodiment 39), characterized in that a test solution of 2.5% w/v of said compound in water is equally or less colored than any one of reference solutions $Y_4$, $BY_4$, $GY_4$, or (optionally) $B_4$; as determined according to Chapter 2.2.2. of the European Pharmacopoeia 6.0. In a sub-embodiment of embodiment 41), the test solution is equally or less colored than reference solutions $GY_4$; as determined according to Chapter 2.2.2. of the European Pharmacopoeia 6.0.

42) Another embodiment relates to the compound of Formula 6 (clazosentan disodium salt); or to the compound according to embodiment 39), characterized in that a test solution of 2.5% w/v of said compound in water is equally or less colored than any one of reference solutions $Y_5$, $BY_5$, $GY_5$, or (optionally) $B_5$; as determined according to Chapter 2.2.2. of the European Pharmacopoeia 6.0. In a sub-embodiment of embodiment 42), the test solution is equally or less colored than reference solutions $GY_5$; as determined according to Chapter 2.2.2. of the European Pharmacopoeia 6.0.

43) Another embodiment relates to the compound of Formula 6 (clazosentan disodium salt); or to the compound according to embodiment 39); characterized in that a test solution of 2.5% w/v of said compound in water is equally or less colored than any one of reference solutions $Y_6$, $BY_6$, $GY_6$, or (optionally) $B_6$; as determined according to Chapter 2.2.2. of the European Pharmacopoeia 6.0. In a sub-embodiment of embodiment 43), the test solution is equally or less colored than reference solutions $GY_6$; as determined according to Chapter 2.2.2. of the European Pharmacopoeia 6.0.

44) Another embodiment relates to the compound of Formula 6 (clazosentan disodium salt); or to the compound according to embodiment 39); characterized in that a test solution of 2.5% w/v of said compound in water is equally or less colored than any one of reference solutions $Y_7$, $BY_7$, $GY_7$, or (optionally) $B_7$; as determined according to Chapter 2.2.2. of the European Pharmacopoeia 6.0. In a sub-embodiment of embodiment 44), the test solution is equally or less colored than reference solutions $GY_7$; as determined according to Chapter 2.2.2. of the European Pharmacopoeia 6.0.

45) Another embodiment relates to a crystalline form of the compound of Formula 6 (clazosentan disodium salt); or to a crystalline form of the compound according to any one of embodiments 39) to 44), characterized by the presence of peaks in the X-ray powder diffractogram at the following angles of refraction 2θ: 9.4°, 12.0°, and 21.9°.

46) Another embodiment relates to a crystalline form of the compound of Formula 6 (clazosentan disodium salt); or to a crystalline form of the compound according to any one of embodiments 39) to 44), characterized by the presence of peaks in the X-ray powder diffractogram at the following angles of refraction 2θ: 9.4°, 12.0°, 18.4°, 21.2°, and 21.9°.

47) Another embodiment relates to a crystalline form of the compound of Formula 6 (clazosentan disodium salt); or to a crystalline form of the compound according to any one of embodiments 39) to 44), characterized by the presence of peaks in the X-ray powder diffractogram at the following angles of refraction 2θ: 9.4°, 12.0°, 13.2°, 17.7°, 18.4°, 19.8°, 21.2°, 21.9°, and 24.9°.

48) Another embodiment relates to a crystalline form of the compound of Formula 6 (clazosentan disodium salt); or to a crystalline form of the compound according to any one of embodiments 39) to 44), characterized

- by an X-ray powder diffractogram with at least four peaks, or notably at least six peaks, or especially at least eight

peaks having an angle of refraction 2θ selected from: 9.4°, 12.0°, 13.2°, 17.7°, 18.4°, 19.8°, 21.2°, 21.9°, and 24.9°; or

- in that an X-ray powder diffractogram essentially shows the pattern as depicted in Figure 1.

For avoidance of any doubt, whenever one of the above embodiments refers to "peaks in the X-ray powder diffractogram at the following angles of refraction 2θ" or "X-ray powder diffractogram with at least...peaks having an angle of refraction 2θ", said X-ray powder diffractogram is obtained by using combined Cu Kα1 and Kα2 radiation, without Kα2 stripping; and it should be understood that the accuracy of the 2θ values as provided herein is in the range of +/- 0.1-0.2°. Notably, when specifying an angle of refraction 2theta (2θ) for a peak in the invention embodiments and the claims, the 2θ value given is to be understood as an interval from said value minus 0.2° to said value plus 0.2° (2θ +/- 0.2°); and especially from said value minus 0.1° to said value plus 0.1° (2θ +/- 0.1°).

When defining the presence of peaks in e.g. an X-ray powder diffractogram, a common approach is to do this in terms of the S/N ratio (S = signal, N = noise). According to this definition, when stating that a peak has to be present in a X-ray powder diffractogram, it is understood that the peak in the X-ray powder diffractogram is defined by having an S/N ratio (S = signal, N = noise) of greater than x (x being a numerical value greater than 1), usually greater than 2, especially greater than 3.

In the context of stating that the crystalline compound essentially shows an X-ray powder diffraction pattern as depicted in, for example, Figure 1, respectively, the term "essentially" means that at least the major peaks of the diffractogram depicted in said figures, i.e. those having a relative intensity of more than 20%, especially more than 10%, as compared to the most intense peak in the diffractogram, have to be present. However, the person skilled in the art of X-ray powder diffraction will recognize that relative intensities in X-ray powder diffractograms may be subject to strong intensity variations due to preferred orientation effects that may result in missing peaks or intensity variations of single peaks.

49) Another aspect relates to the compound of Formula 6 (clazosentan disodium salt) according to any one of embodiments 40) to 48); or to the compound according to embodiment 39), characterized by an assay of at least 90% w/w, at least 91% w/w, at least 92% w/w; at least 93% w/w, at least 94% w/w, at least 95% w/w, at least 96% w/w, at least 97% w/w, at least 98% w/w, or at least 99% w/w (especially an assay of 99.1%) , as determined by high-performance liquid chromatography (HPLC) [by the HPLC Method B as described herein or by an analogous HPLC method].

The term "assay" refers to a common in the pharmaceutical industry quantitative measure of the amount of an analyte in a sample. In particular, the term, as used herein, refers the amount of the compound of Formula 6 in a sample, expressed as a percentage of the total weight of the sample (% w/w). Assay (assay "as is") is notably measured by HPLC, especially by the HPLC Method B as disclosed herein.

50) Another aspect relates to the compound of Formula 6 (clazosentan disodium salt) according to any one of embodiments 40) to 49); or to the compound according to embodiment 39), wherein the compound of Formula 6 comprises a total amount of impurities of not more than 5% w/w, not more than 4% w/w, notably not more than 3% w/w, especially not more than 2% w/w, wherein the impurities are determined by high-performance liquid chromatography (HPLC) [by the HPLC Method B as described herein or by an analogous HPLC method].

The term "impurity" as used herein refers to the amount of specified and unspecified impurity (also referred to specified and unspecified related substance) detectable by HPLC, especially by the HPLC Method B as disclosed herein. The total amount of impurities as mentioned herein refers to the sum of essentially all specified and unspecified impurities detectable by HPLC, especially by the HPLC Method B as disclosed herein.

51) Another aspect relates to the compound of Formula 6 (clazosentan disodium salt) according to any one of embodiments 40) to 50); or to the compound according to embodiment 39), wherein the compound of Formula 6 comprises a total amount of residual solvents of not more than 5% w/w, not more than 4% w/w, notably not more than 3% w/w, especially not more than 2% w/w, in particular not more than 1% w/w, wherein the solvents are selected from a group consisting of ethylene glycol, ethanol, methanol, tetrahydrofuran, and dimethylformamide (wherein the total amount of residual solvents is determined by gas chromatography; notably headspace gas chromatography (GC-HS) as described herein).

For avoidance of doubt, the total amount of residual solvents refers to the sum of the individual amounts of ethylene glycol, ethanol, methanol, tetrahydrofuran, and dimethylformamide; determined in a sample.

52) Another aspect relates to the compound of Formula 6 (clazosentan disodium salt) according to any one of embodiments 40) to 51); or to the compound according to embodiment 39), wherein the compound of Formula 6 comprises not more than 0.5% w/w of a residual solvent selected from ethanol (as determined by gas chromatography; notably headspace gas chromatography (GC-HS) as described herein).

53) Another aspect relates to the compound of Formula 6 (clazosentan disodium salt) according to any one of embodiments 40) to 52); or to the compound according to embodiment 39), wherein the compound of Formula 6 comprises not more than 0.3% w/w of a residual solvent selected from methanol (as determined by gas chromatography; notably headspace gas chromatography (GC-HS) as described herein).

54) Another aspect relates to the compound of Formula 6 (clazosentan disodium salt) according to any one of embodiments 40) to 53); or to the compound according to embodiment 39), wherein the compound of Formula 6 comprises not more than 0.05% w/w of a residual solvent selected from tetrahydrofuran (as determined by gas chromatography; notably headspace gas chromatography (GC-HS) as described herein).

55) Another aspect relates to the compound of Formula 6 (clazosentan disodium salt) according to any one of embodiments 40) to 54); or to the compound according to embodiment 39), wherein the compound of Formula 6 comprises not more than 0.05% w/w of a residual solvent selected from dimethylformamide (as determined by gas chromatography; notably headspace gas chromatography (GC-HS) as described herein).

56) Another aspect relates to the compound of Formula 6 (clazosentan disodium salt) according to any one of embodiments 40) to 55); or to the compound according to embodiment 39), wherein the compound of Formula 6 comprises not more than 0.062% w/w of a residual solvent selected from ethylene glycol (as determined by gas chromatography; notably headspace gas chromatography (GC-HS) as described herein).

It is understood that any one of embodiments 40) to 56) refers to two alternatives, namely, the compound of Formula 6 (clazosentan disodium salt) *per se* and to the compound according to embodiment 39) [or according to embodiment 39) to 44) and 49) to 56)] i.e. the compound of Formula 6, obtainable by the corresponding processes; wherein each of said alternatives is independently further characterized by the respective technical features such as angles of refraction, coloration, assay, total amount of impurities, amount of residual solvents, etc.

57) Another aspect relates to a pharmaceutical composition (notably for intravenous infusion; especially solution for intravenous infusion) comprising a compound according to any one of embodiments 39) to 56), wherein said composition further comprises at least one pharmaceutically acceptable carrier (especially water) and optionally at least one pharmaceutically acceptable excipient. Notably, such pharmaceutical composition comprises a compound according to any one of embodiments 39) to 56), optionally a buffer (e.g. tris(hydroxymethyl)aminomethane) (TRIS), optionally a chelating agent (e.g. disodium edetate), optionally an isotonic agent (e.g. sodium chloride), and water.

58) Another embodiment relates to a pharmaceutical composition (notably for intravenous infusion; especially such composition is a solution for intravenous infusion) according to embodiment 57), wherein said composition comprises

- from about 20 mg/mL to about 40 mg/mL of compound according to any one of embodiments 39) to 56);
- water;
- optionally from about 5 mg/mL to about 15 mg/mL tris(hydroxymethyl)aminomethane;
- optionally from about 0.05 to about 0.15 mg/mL disodium edetate; and
- optionally from about 2.0 to about 3.0 mg/mL sodium chloride.

59) Another embodiment relates to a pharmaceutical composition (notably for intravenous infusion; especially such composition is a solution for intravenous infusion) according to according to embodiment 57), wherein said composition comprises

- from about 25.0 mg/mL to about 29.0 mg/mL (especially about 27.0 mg/mL) of the compound according to any one of embodiments 39) to 56);
- water;
- optionally from about 8.0 mg/mL to about 12.0 mg/mL (especially about 10.0 mg/mL) tris(hydroxymethyl)aminomethane;
- optionally from about 0.08 mg/mL to about 0.12 mg/mL (especially about 0.1 mg/mL) disodium edetate; and
- optionally from about 2.0 mg/mL to about 3.0 mg/mL (especially about 2.5 mg/mL) sodium chloride.

It is understood that the amount of the compound of Formula 6 disodium salt in the pharmaceutical compositions according to the present invention can be calculated based on the amount of the compound of Formula 6 free acid present in said compositions, wherein the amount of the compound of Formula 6 free acid can be directly determined by common in the art analytical methods such as HPLC. For example, it is understood that from about 25.0 mg/mL to about 29.0 mg/mL (or about 27.0 mg/mL) of the compound of Formula 6 disodium salt corresponds to from about 23.2 to about 26.9 (or about 25.0 mg/mL) of the compound of Formula 6 free acid, wherein the amount of the compound of Formula 6 free acid may be directly determined by HPLC. The term "compound of Formula 6 free acid" as used herein refers to the compound of Formula 5.

60) Another embodiment relates to a pharmaceutical composition (notably for intravenous infusion; especially such composition is a solution for intravenous infusion) according to according to embodiment 57), wherein said composition comprises

- from about 23.5 mg/mL to about 26.5 mg/mL (especially about 25 mg/mL) of the compound according to any one of

embodiments 39) to 56), wherein said concentration of said compound (given in mg/mL) refers to the compound according to any one of embodiments 39) to 56) in its free acid form;

- from about 8.0 mg/mL to about 12.0 mg/mL (especially about 10.0 mg/mL) tris(hydroxymethyl)aminomethane;
- from about 0.08 mg/mL to about 0.12 mg/mL (especially about 0.1 mg/mL) disodium edetate; and
- from about 2.0 mg/mL to about 3.0 mg/mL (especially about 2.5 mg/mL) sodium chloride.

61) Another embodiment relates to a pharmaceutical composition according to any one of embodiments 59) or 60), wherein said composition is equally or less colored than any one of reference solutions $Y_3$, $BY_3$, $GY_3$, or (optionally) $B_3$; as determined according to Chapter 2.2.2. of the European Pharmacopoeia 6.0.

62) Another embodiment relates to a pharmaceutical composition according to any one of embodiments 59) or 60), wherein said composition is equally or less colored than reference solution $GY_3$, as determined according to Chapter 2.2.2. of the European Pharmacopoeia 6.0.

It is understood that the pharmaceutical compositions according to the present invention may further comprise hydrochloric acid such that the pH value of said compositions is from about 7.0 to about 9.0; notably from about 7.5 to about 8.5; especially 8.0 $\pm$ 0.1.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the crystalline form of the present invention, optionally in combination with other therapeutically valuable substances, into a galenical administration form together with suitable, non-toxic, inert, pharmaceutically acceptable solid or liquid carrier materials and, if desired, usual pharmaceutical adjuvants. Examples of pharmaceutically acceptable excipients are *inter alia* co-solvents, buffers, metal ion chelating agents, surfactants, osmolarity regulators, preservatives, viscosity modulators and solubilizers.

It is understood that the crystalline forms according to the present invention may comprise non-coordinated and / or coordinated solvent. Coordinated solvent is used herein as term for a crystalline solvate (e.g. hydrate). Likewise, non-coordinated solvent is used herein as term for physiosorbed or physically entrapped solvent (definitions according to Polymorphism in the Pharmaceutical Industry (Ed. R. Hilfiker, VCH, 2006), Chapter 8: U.J. Griesser: The Importance of Solvates). The crystalline form according to any one of embodiments 45) to 49) corresponds to a non-solvated, non-hydrated crystalline form, but may comprise non-coordinated water or another non-coordinated solvent.

The crystalline form according to the present invention decomposes at about 250°C as measured by DSC.

It is understood that the sodium content in the compound of Formula 6 as disclosed herein is about 2 equivalents. The sodium content of said compound may vary within the normal technical limits acceptable in the pharmaceutical industry. Notably, the sodium content of the compound of Formula 6 as disclosed herein may be from about 6.4% w/w to about 8.4% w/w of the total weight; especially from about 7.2% w/w to about 7.7% w/w; in particular about 7.4% w/w; as determined by common in the art methods such as potentiometric titration according to USP <541> / Ph. Eur. 2.2.20. Ph. Eur. 2.2.20 refers to Chapter 2.2.20 of the European pharmacopeia 6.0. Potentiometric titration may be performed by the method described herein.

63) Another aspect relates to the use of the compound of Formula 6 according to any one of embodiments 39) to 56) for the manufacturing of a pharmaceutical composition; especially a pharmaceutical composition according to any one of embodiments 57) to 62).

64) Another aspect relates to the compound of Formula 6 according to any one of embodiments 39) to 56), or a composition according to any one of embodiments 57) to 62), for use as a medicament.

65) Another embodiment relates to the compound of Formula 6 according to any one of embodiments 39) to 56), or a composition according to any one of embodiments 57) to 62), for use in the prevention or treatment of a disease or disorder where endothelin receptors (especially $ET_A$-receptors) are involved.

66) Another embodiment relates to the compound of Formula 6 according to any one of embodiments 39) to 56), or a composition according to any one of embodiments 57) to 62), for use in the prevention or treatment (especially prevention) of cerebral vasospasm, and/or vasospasm-related cerebral infarction and cerebral ischemic symptoms after aneurysmal subarachnoid hemorrhage surgery.

67) Another embodiment relates to the compound of Formula 6 according to any one of embodiments 39) to 56), or a composition according to any one of embodiments 57) to 62), for use in the prevention or treatment (especially prevention) of (vasospasm-related and all-cause) morbidity and (all-cause) mortality (especially vasospasm-related morbidity and all-cause mortality) in patients after aneurysmal subarachnoid hemorrhage surgery.

Aneurysmal subarachnoid hemorrhage surgery (or aneurysm repair) as mentioned herein may especially be performed by surgical clipping or endovascular coiling. The term "aneurysmal subarachnoid hemorrhage surgery", as used herein, also refers to aneurysmal subarachnoid hemorrhage securing.

68) Another embodiment relates to the compound of Formula 6 according to any one of embodiments 39) to 56), or a composition according to any one of embodiments 57) to 62), for use in the prevention or treatment (especially prevention) of cerebral vasospasm, and/or vasospasm-related cerebral infarction and cerebral ischemic symptoms after

aneurysmal subarachnoid hemorrhage surgery, wherein a pharmaceutical composition comprising the compound is administered to a patient as intravenous continuous infusion at a dosage of about 5 mg/h, about 10 mg/h, or about 15 mg/h (notably about 10 mg/h or about 15 mg/h; especially about 10 mg/h), wherein said dosage refers to the amount of the compound in its free acid form [wherein said administration starts after surgery of aneurysmal subarachnoid hemorrhage and continues for up to 15 days after aneurysmal rupture].

69) Another embodiment relates to the compound of Formula 6 according to any one of embodiments 39) to 56), or a composition according to any one of embodiments 57) to 62), for use in the prevention or treatment (especially prevention) of complications and/or permanent brain damage related to cerebral vasospasm in patients (especially in patients with thick and diffuse cloth) following aneurysmal subarachnoid hemorrhage.

70) Another embodiment relates to the compound of Formula 6 according to any one of embodiments 39) to 56), or a composition according to any one of embodiments 57) to 62), for use in the reduction of the incidence and/or severity of cerebral vasospasm in patients (especially in patients with thick and diffuse cloth) following aneurysmal subarachnoid hemorrhage.

71) Another embodiment relates to the compound of Formula 6 according to any one of embodiments 39) to 56), or a composition according to any one of embodiments 57) to 62), for use in the reduction of the incidence and/or severity of cerebral infarction in patients (especially in patients with thick and diffuse cloth) following aneurysmal subarachnoid hemorrhage.

72) Another embodiment relates to the compound of Formula 6 according to any one of embodiments 39) to 56), or a composition according to any one of embodiments 57) to 62), for use the prevention or treatment (especially prevention) of cerebral infarction due to vasospasm-related delayed cerebral ischemia in patients (especially in patients with thick and diffuse cloth) following aneurysmal subarachnoid hemorrhage.

73) Another embodiment relates to the compound of Formula 6 according to any one of embodiments 39) to 56), or a composition according to any one of embodiments 57) to 62), for use in the prevention or treatment (especially prevention) of clinical deterioration due to vasospasm-related delayed cerebral ischemia in patients (especially in patients with thick and diffuse cloth) following aneurysmal subarachnoid hemorrhage.

74) Another embodiment relates to the compound of Formula 6 according to any one of embodiments 39) to 56), or a composition according to any one of embodiments 57) to 62), for use in the prevention or treatment (especially prevention) of cerebral vasospasm in patients (especially in patients with thick and diffuse cloth) following aneurysmal subarachnoid hemorrhage.

75) Another embodiment relates to the compound of Formula 6 according to any one of embodiments 39) to 56), or a composition according to any one of embodiments 57) to 62), for use in the prevention or treatment (especially prevention) of (notably vasospasm-related or all-cause; especially vasospasm-related) morbidity and/or (notably vasospasm-related or all-cause; especially vasospasm-related) mortality in patients (especially in patients with thick and diffuse cloth) following aneurysmal subarachnoid hemorrhage.

76) Another embodiment relates to the compound of Formula 6 according to any one of embodiments 39) to 56), or a composition according to any one of embodiments 57) to 62), for use in any of the following: reducing the duration of the hospitalization/duration in an intensive care unit of patients (especially in patients with thick and diffuse cloth); reducing the time to return to work for patients (especially in patients with thick and diffuse cloth) following aneurysmal subarachnoid hemorrhage; reducing the need/amount of rehabilitation in patients (especially in patients with thick and diffuse cloth) following aneurysmal subarachnoid hemorrhage; improving (long-term) clinical outcome in patients (especially in patients with thick and diffuse cloth) following aneurysmal subarachnoid hemorrhage; and/or improving cognition/cognitive function/quality of life in patients (especially in patients with thick and diffuse cloth) following aneurysmal subarachnoid hemorrhage.

77) Another embodiment relates to the compound of Formula 6 to any one of embodiments 39) to 56), or a composition according to any one of embodiments 57) to 62), for use according to any one of embodiments 65) to 76), wherein a pharmaceutical composition comprising the compound is administered to a patient (especially in a patient with thick and diffuse cloth) as intravenous continuous infusion at a dosage of about 5 mg/h, about 10 mg/h, or about 15 mg/h (notably about 10 mg/h or about 15 mg/h; especially about 15 mg/h), wherein said dosage refers to the amount of the compound in its free acid form [wherein said administration starts following the time of the aneurysm rupture and continues for up to 14 days thereafter].

For avoidance of doubt, any reference to the compound of Formula 6 according to any one of embodiments 39) to 56), or a composition according to any one of embodiments 57) to 62), for use in the prevention or treatment of any disease or disorder according to embodiments 65) to 76) also discloses a method of prevention or treatment (especially prevention) of said disease or disorder, said method comprising administering to a subject in need thereof (an effective amount of) the compound of Formula 6 according to any one of embodiments 39) to 56).

For avoidance of doubt, any reference to the compound of Formula 6 according to any one of embodiments 39) to 56), or a composition according to any one of embodiments 57) to 62), for use in the prevention or treatment of any disease or disorder according to embodiments 65) to 76) also discloses the use of the compound of Formula 6 according to any one

of embodiments 39) to 49), in the preparation of a medicament for the prevention or treatment of said disease or disorder.

For avoidance of doubt, any reference to the compound of Formula 6 according to any one of embodiments 39) to 49), or a composition according to any one of embodiments 49) to 55), for use in the prevention or treatment of any disease or disorder according to embodiments 65) to 76) also discloses the compound of Formula 6 according to any one of embodiments 39) to 56), for use in a method of prevention or treatment of said disease or disorders.

For avoidance of doubt, any reference to the compound of Formula 6 according to any one of embodiments 39) to 56), or a composition according to any one of embodiments 57) to 62), for use in the prevention or treatment of any disease or disorder according to embodiments 65) to 76) also discloses a medicament for the prevention or treatment (especially prevention) of said disease or disorder, said medicament comprising the compound of Formula 6 according to any one of embodiments 39) to 56).

The term "effective amount" as used herein refers to a dose from about 5 mg/day to about 20 mg/day (especially about 5 mg/day, about 10 mg/day, or about 15 mg/day) administered to a subject for up to 15 days (especially for up to 14). The effective amount is preferably administered intravenously preferably intravenously as a continuous infusion.

The term "thick and diffuse cloth" as used herein refers to a thick confluent clot, more than 4 mm in thickness, involving 3 or more basal cisterns, as measured by computed tomography (CT). Patients diagnosed with thick and diffuse cloth may be classified as high-risk patients in which the use of the compound of the present invention may be particularly indicated.

The term "cerebral infarction" as used herein refers to all-cause new or worsened cerebral infarction (notably said infraction having a total volume of more than about 5 $cm^3$). New or worsened cerebral infarctions may be determined by central radiology review comparing CT scans performed 16 days after initiation of therapy with a drug with the CT scan performed before said initiation of therapy.

The term "clinical deterioration" as used herein refers to a worsening of at least two points compared to the reference score, on the mGCS or the aNIHSS scales, lasting for at least 2 hours, which cannot be entirely attributed to causes other than cerebral vasospasm. Clinical deterioration due to delayed cerebral ischemia may be adjudicated based on review of clinical data, case narratives, angiograms and/or CT scans. The term "mGCS" refers to the Glasgow Coma Scale, which is a neurological scale aiming to give a reliable and objective way of recording the state of a person’s consciousness. The term "aNIHSS" refers to the abbreviated National Institutes of Health Stroke Scale: a tool used by healthcare providers to objectively quantify the impairment caused by a stroke.

The terms "subject", and likewise, "patient" refers to mammals, especially humans. Preferably, the term "subject" refers to the term "patient".

Based on the dependencies of the different embodiments 1) to 6) as disclosed hereinabove, the following embodiments are thus possible and intended and herewith specifically disclosed in individualized form: 2+1, 3+2+1, 4+1, 4+2+1, 4+3+2+1, 5+1, 5+2+1, 5+3+2+1, 5+4+1, 5+4+2+1, 5+4+3+2+1, 6+1, 6+2+1, 6+3+2+1, 6+4+1, 6+4+2+1, 6+4+3+2+1, 6+5+1, 6+5+2+1, 6+5+3+2+1, 6+5+4+1, 6+5+4+2+1, or 6+5+4+3+2+1.

Based on the dependencies of the different embodiments 7) to 11) as disclosed hereinabove, the following embodiments are thus possible and intended and herewith specifically disclosed in individualized form: 8+7, 9+7, 9+8+7, 10+7, 10+8+7, 10+9+7, 10+9+8+7, 11+7, 11+8+7, 11+9+7, 11+9+8+7, 11+10+7, 11+10+8+7, 11+10+9+7, or 11+10+9+8+7.

Based on the dependencies of the different embodiments 12) to 18) as disclosed hereinabove, the following embodiments are thus possible and intended and herewith specifically disclosed in individualized form: 13+12, 14+12, 14+13+12, 15+12, 15+13+12, 15+14+12, 15+14+13+12, 16+12, 16+13+12, 16+14+12, 16+14+13+12, 16+15+12, 16+15+13+12, 16+15+14+12, 16+15+14+13+12, 17+12, 17+13+12, 17+14+12, 17+14+13+12, 17+15+12, 17+15+13+12, 17+15+14+12, 17+15+14+13+12, 17+16+12, 17+16+13+12, 17+16+14+12, 17+16+14+13+12, 17+16+15+12, 17+16+15+13+12, 17+16+15+14+12, 17+16+15+14+13+12, 18+12, 18+13+12, 18+14+12, 18+14+13+12, 18+15+12, 18+15+13+12, 18+15+14+12, 18+15+14+13+12, 18+16+12, 18+16+13+12, 18+16+14+12, 18+16+14+13+12, 18+16+15+12, 18+16+15+13+12, 18+16+15+14+12, 18+16+15+14+13+12, 18+17+12, 18+17+13+12, 18+17+14+12, 18+17+14+13+12, 18+17+15+12, 18+17+15+13+12, 18+17+15+14+12, 18+17+15+14+13+12, 18+17+16+12, 18+17+16+13+12, 18+17+16+14+12, 18+17+16+14+13+12, 18+17+16+15+12, 18+17+16+15+13+12, or 18+17+16+15+14+12, 18+17+16+15+14+13+12.

Based on the dependencies of the different embodiments 19) to 24) as disclosed hereinabove, the following embodiments are thus possible and intended and herewith specifically disclosed in individualized form: 20+19, 21+20+19, 22+19, 22+20+19, 22+21+20+19, 23+19, 23+20+19, 23+21+20+19, 23+22+19, 23+22+20+19, 23+22+21+20+19, 24+19, 24+20+19, 24+21+20+19, 24+22+19, 24+22+20+19, 24+22+21+20+19, 24+23+19, 24+23+20+19, 24+23+21+20+19, 24+23+22+19, 24+23+22+20+19, or 24+23+22+21+20+19.

Based on the dependencies of the different embodiments 25) to 28) as disclosed hereinabove, the following embodiments are thus possible and intended and herewith specifically disclosed in individualized form: 26+25, 27+25, 27+26+25, 28+25, 28+26+25, 28+27+25, or 28+27+26+25.

Based on the dependencies of the different embodiments 39) to 60) as disclosed hereinabove, the following

embodiments are thus possible and intended and herewith specifically disclosed in individualized form: 45+39, 45+40, 45+41, 45+42, 45+43, 45+44, 46+39, 46+40, 46+41, 46+42, 46+43, 46+44, 47+39, 47+40, 47+41, 47+42, 47+43, 47+44, 48+39, 48+40, 48+41, 48+42, 48+43, 48+44, 49+39, 49+40, 49+41, 49+42, 49+43, 49+44, 49+45+39, 49+45+40, 49+45+41, 49+45+42, 49+45+43, 49+45+44, 49+46+39, 49+46+40, 49+46+41, 49+46+42, 49+46+43, 49+46+44, 49+47+39, 49+47+40, 49+47+41, 49+47+42, 49+47+43, 49+47+44, 49+48+39, 49+48+40, 49+48+41, 49+48+42, 49+48+43, 49+48+44, 50+39, 50+40, 50+41, 50+42, 50+43, 50+44, 50+45+39, 50+45+40, 50+45+41, 50+45+42, 50+45+43, 50+45+44, 50+46+39, 50+46+40, 50+46+41, 50+46+42, 50+46+43, 50+46+44, 50+47+39, 50+47+40, 50+47+41, 50+47+42, 50+47+43, 50+47+44, 50+48+39, 50+48+40, 50+48+41, 50+48+42, 50+48+43, 50+48+44, 51+39, 51+40, 51+41, 51+42, 51+43, 51+44, 51+45+39, 51+45+40, 51+45+41, 51+45+42, 51+45+43, 51+45+44, 51+46+39, 51+46+40, 51+46+41, 51+46+42, 51+46+43, 51+46+44, 51+47+39, 51+47+40, 51+47+41, 51+47+42, 51+47+43, 51+47+44, 51+48+39, 51+48+40, 51+48+41, 51+48+42, 51+48+43, 51+48+44, 52+39, 52+40, 52+41, 52+42, 52+43, 52+44, 52+45+39, 52+45+40, 52+45+41, 52+45+42, 52+45+43, 52+45+44, 52+46+39, 52+46+40, 52+46+41, 52+46+42, 52+46+43, 52+46+44, 52+47+39, 52+47+40, 52+47+41, 52+47+42, 52+47+43, 52+47+44, 52+48+39, 52+48+40, 52+48+41, 52+48+42, 52+48+43, 52+48+44, 53+39, 53+40, 53+41, 53+42, 53+43, 53+44, 53+45+39, 53+45+40, 53+45+41, 53+45+42, 53+45+43, 53+45+44, 53+46+39, 53+46+40, 53+46+41, 53+46+42, 53+46+43, 53+46+44, 53+47+39, 53+47+40, 53+47+41, 53+47+42, 53+47+43, 53+47+44, 53+48+39, 53+48+40, 53+48+41, 53+48+42, 53+48+43, 53+48+44, 54+39, 54+40, 54+41, 54+42, 54+43, 54+44, 54+45+39, 54+45+40, 54+45+41, 54+45+42, 54+45+43, 54+45+44, 54+46+39, 54+46+40, 54+46+41, 54+46+42, 54+46+43, 54+46+44, 54+47+39, 54+47+40, 54+47+41, 54+47+42, 54+47+43, 54+47+44, 54+48+39, 54+48+40, 54+48+41, 54+48+42, 54+48+43, 54+48+44, 55+39, 55+40, 55+41, 55+42, 55+43, 55+44, 55+45+39, 55+45+40, 55+45+41, 55+45+42, 55+45+43, 55+45+44, 55+46+39, 55+46+40, 55+46+41, 55+46+42, 55+46+43, 55+46+44, 55+47+39, 55+47+40, 55+47+41, 55+47+42, 55+47+43, 55+47+44, 55+48+39, 55+48+40, 55+48+41, 55+48+42, 55+48+43, 55+48+44, 56+39, 56+40, 56+41, 56+42, 56+43, 56+44, 56+45+39, 56+45+40, 56+45+41, 56+45+42, 56+45+43, 56+45+44, 56+46+39, 56+46+40, 56+46+41, 56+46+42, 56+46+43, 56+46+44, 56+47+39, 56+47+40, 56+47+41, 56+47+42, 56+47+43, 56+47+44, 56+48+39, 56+48+40, 56+48+41, 56+48+42, 56+48+43, 56+48+44, 59+39, 59+40, 59+41, 59+42, 59+43, 59+44, 59+45+39, 59+45+40, 59+45+41, 59+45+42, 59+45+43, 59+45+44, 59+46+39, 59+46+40, 59+46+41, 59+46+42, 59+46+43, 59+46+44, 59+47+39, 59+47+40, 59+47+41, 59+47+42, 59+47+43, 59+47+44, 59+48+39, 59+48+40, 59+48+41, 59+48+42, 59+48+43, 59+48+44, 60+39, 60+40, 60+41, 60+42, 60+43, 60+44, 60+45+39, 60+45+40, 60+45+41, 60+45+42, 60+45+43, 60+45+44, 60+46+39, 60+46+40, 60+46+41, 60+46+42, 60+46+43, 60+46+44, 60+47+39, 60+47+40, 60+47+41, 60+47+42, 60+47+43, 60+47+44, 60+48+39, 60+48+40, 60+48+41, 60+48+42, 60+48+43, or 60+48+44.

In the list above the numbers refer to the embodiments according to their numbering provided hereinabove whereas "+" indicates the dependency from another embodiment. The different individualized embodiments are separated by commas. In other words, "5+2+1" for example refers to embodiment 5) depending on embodiment 2), depending on embodiment 1), i.e. embodiment "5+2+1" corresponds to embodiment 5) further characterized by the features of the embodiments 2) and 1).

Definitions provided herein are intended to apply uniformly to the subject matter as defined in any one of the embodiments disclosed herein, and, *mutatis mutandis,* throughout the description and the claims unless an otherwise expressly set out definition provides a broader or narrower definition. It is well understood that a definition or preferred definition of a term or expression defines and may replace the respective term or expression independently of (and in combination with) any definition or preferred definition of any or all other terms or expressions as defined herein.

Where the plural form is used for compounds, solids, pharmaceutical compositions, diseases, and the like, this is intended to also mean a single compound, solid, pharmaceutical composition, disease, or the like.

Unless used regarding temperatures, the term "about" placed before a numerical value "X" refers in the current application to an interval extending from X minus 10% of X to X plus 10% of X, and preferably to an interval extending from X minus 5% of X to X plus 5% of X; most preferred is X. In the particular case of temperatures, the term "about" placed before a temperature "Y" refers in the current application to an interval extending from the temperature Y minus 10°C to Y plus 10°C, preferably to an interval extending from Y minus 5°C to Y plus 5°C. Room temperature means a temperature of about 25°C.

Whenever the word "between" or "to" is used to describe a numerical range, it is to be understood that the end points of the indicated range are explicitly included in the range. For example: if a temperature range is described to be between 40°C and 80°C (or 40°C to 80°C), this means that the end points 40°C and 80°C are included in the range; or if a variable is defined as being e.g. an integer between 1 and 7 (or 1 to 7), this means that the variable is the integer 1, 2, 3, 4, 5, 6 or 7.

The term "solvate(s)" either used alone or in combination is understood in the context of the present invention to mean an aggregate comprising a compound or a salt thereof as defined herein and one or more molecules of a solvent. Hydrates are a special form of solvates, wherein the one or more solvent molecules comprised in said aggregate are water molecules.

The compound of Formula 6 in its form as disclosed herein may be present in different isomeric/tautomeric forms

with respect to the position of the sodium atom in the tetrazole ring and/or the sulphonamide group as depicted below:

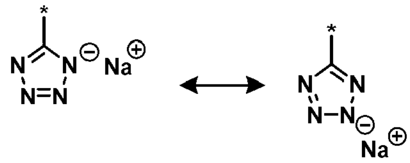

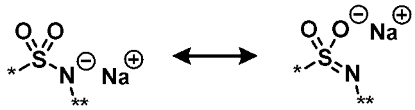

It is thus understood that all possible isomers/tautomers of the compound of Formula 6 in its solid form are within the scope of the present invention, even though only one isomer/tautomer of the compound of Formula 6 may be depicted herein. In solution, such compounds exist usually as mixtures of different isomeric/tautomeric forms; in the solid state typically one form predominates. The asterisks "*" and "**" in the formulas hereinabove denote the point of attachment of the corresponding group to the rest of the molecule of the compound of Formula 6. By analogy, within the scope of the present invention are all possible isomeric/tautomeric forms of the compounds of Formula 2a and 3a with respect to the sulphonamide group.

The terms "w/w" (weight per weight), "w/v" (weight per volume), "v/v" (volume per volume) refer to concentration i.e. the proportion of a particular substance within a mixture, as measured by weight/volume per weight/volume. When expressed in percentage such as "% w/w" , "% w/v", or % "v/v" the terms refer to the corresponding percentage concentration.

The term "vol." refers to volumes (in L, e.g. of a solvent) per unit weight (in kg, e.g. of a reactant). For example, 1 vol. refers to 1 liter (e.g. of a solvent) per kg (e.g. of a reactant).

The term "wt." refers weight (e.g. in g) of a compound (e.g. a reactant) A per weight (e.g. in g) of a compound B (e.g. a reactant). For example, 1 wt. of a compound A per 5 g of a compound B means 5 g of a compound A; also, a mixture of 1 wt. methanol and 3 wt. ethanol means that the weight ratio methanol:ethanol in the mixture is 1:3.

The term "equivalent(s)", also abbreviated "eq.", refers to the number of moles of a compound that reacts with (or is equivalent to) an arbitrary number of moles of another compound in a given chemical reaction.

**Abbreviations** (as used hereinbefore or hereinafter):

| | |
|---|---|
| a/a | Area/area |
| ACN | Acetonitrile |
| aq. | Aqueous |
| d | Day or days |
| DABCO | 1,4-Diazabicyclo[2.2.2]octane |
| DBU | 1,8-Diazabicyclo[5.4.0]undec-7-ene |
| DMF | dimethylformamide |
| DMSO | dimethyl sulfoxide |
| BHT | 2,6-Di-*tert*-butyl-4-methylphenol |
| eq. | equivalent(s) |
| EDTA | Ethylenediaminetetraacetic acid |
| EtOH | Ethanol |
| eq. | Equivalent(s) |
| h | Hour(s) |
| $^1$H-NMR | Proton nuclear magnetic resonance |
| HPLC | High performance liquid chromatography |
| IPC | In-Process Control |
| $iPr_2NEt$ | N,N-Diisopropylethylamine |
| KOtBu | Potassium *tert*-butylate |
| MeOH | Methanol |
| min | Minute(s) |
| ml or mL | Milliliter(s) |

| | |
|---|---|
| NaOMe | Sodium methoxide |
| $NEt_3$ | Triethylamine |
| 2-PrOH | Isopropyl alcohol |
| r.t. | Room temperature |
| THF | Tetrahydrofuran |
| TMS-Cl | Trimethylsilyl chloride |
| TMS-CN | Trimethylsilyl cyanide |
| UV | Ultraviolet |

## Experimental Part

All temperatures are stated in °C.

### HPLC

#### Method A

**Equipment:** Agilent 1100 series system with online degasser (e.g. G1322A), low-pressure quaternary pump (e.g. G1311A), autosampler (e.g. G1329A), temperature-controlled column compartment (e.g. G1316A), and UV detector (e.g. DADG1315B) or equivalent. **Solvent:** ACN/$H_2O$ = 3/1 or ACN/$H_2O$ = 1/1 (depending on sample, addition of DMSO may be necessary). **Column:** Halo C18 4.6 mm x 100 mm, 2.7 μm. **Mobile phase:** A: 0.05% (v/v) HCOOH in water, B: 0.05% (v/v) HCOOH in ACN. **Column temperature:** 40.0 ± 1.0°C. **Data collection time:** 12 min. Flow rate: 1.5 mL/min. **Gradient:**

| Time [min] | A [%] | B [%] |
|---|---|---|
| 0 | 97 | 3 |
| 7 | 3 | 97 |
| 10 | 3 | 97 |
| 10.1 | 97 | 3 |
| 12 | 97 | 3 |

#### Method B

**Equipment:** Agilent 1100/1200/1260 series system with online degasser (e.g. G1322A), low-pressure quaternary pump (e.g. G1311A), autosampler (e.g. G1329A), temperature-controlled column compartment (e.g. G1316A), and UV detector (e.g. DADG1315B) or equivalent. **Solvent:** mobile phase A. **Column:** Waters X-Bridge C18, 150 mm x 3.0 mm, 3.5 μm. **Mobile phase:** A: Buffer (3.7 mM sodium tetraborate decahydrate pH 9.0) / ACN / tetrabutylammonium hydroxide 70/30/0.35 (v/v/v) and B: Buffer / ACN / tetrabutylammonium hydroxide 40/60/0.35 (v/v/v). **Column temperature:** 50°C. **Data collection time:** 15 min. Flow rate: 1.0 mL/min. **Gradient:**

| Time [min] | A [%] | B [%] |
|---|---|---|
| 0 | 100 | 0 |
| 6 | 60 | 40 |
| 8 | 0 | 100 |
| 8.1 | 100 | 0 |
| 15 | 100 | 0 |

The assay value (assay "as is") may be calculated in accordance with the following formula:

$$Assay, as\ is\ [\%\,w/w] = \frac{PA_{SPL}}{PA_{STD}} \times \frac{W_{STD}}{W_{SPL}} \times P_{STD}$$

,wherein $PA_{SPL}$ refers to the peak area in the HPLC of the compound of Formula 6 in the sample solution, given in [mAU*min]; $PA_{STD}$ refers to the peak area of the compound of Formula 6 in all working standards in the sequence [mAU*min]; $W_{STD}$ refers to the weight of the working standard [mg]; $W_{SPL}$ refers to the sample weight [mg]; $P_{STD}$ refers to the potency of the reference standard [%]. $P_{STD}$ may be calculated in accordance with the following formula:

$$P_{STD} = (100 - solvents - water) \times \frac{Purity}{100}$$ ,

wherein "solvents" refers to the sum of all residual solvents for the reference material [% w/w]; "Water" refers to the water content determined for the reference material (e.g. by Karl Fischer titration (method described herein)) [%w/w]; Purity refers to the purity of the reference material [%a/a].

The assay value referred to in the present application represent a value which has been corrected for residual solvents and water, which may be present in the sample for analysis. Residual solvents are ethylene glycol, tetrahydrofuran, methanol, ethanol, and N,N-dimethylformamide. The correction is performed by first determining the amount of residual solvents (by gas chromatography (method described herein)) and the amount of water (by Karl Fischer titration (method described herein)) in the sample, and then calculating the assay value (i.e. anhydrous and solvent free assay) according to the following formula:

$$Assay, anhydrous, solvent\ free\ [\%\ w/w] = \frac{PA_{SPL}}{PA_{STD}} \times \frac{W_{STD}}{W_{SPL}} \times \frac{P_{STD}}{(100 - water - solvents)} \times 100\%$$ ,

wherein $PA_{SPL}$ refers to the peak area in the HPLC of the compound of Formula 6, given in [mAU*min]; $PA_{STD}$ refers to the peak area of the compound of Formula 6 in all working standards in the sequence [mAU*min]; $W_{STD}$ refers to the weight of the working standard [mg]; $W_{SPL}$ refers to the sample weight [mg]; $P_{STD}$ refers to the potency of the reference standard [%] and is calculated in accordance with the formula mentioned hereinabove; "Solvents" refers to the sum of the residual solvents of a sample [% w/w]. Residual solvents are ethylene glycol, tetrahydrofuran, methanol, ethanol, and N,N-dimethylformamide. "Water" refers to the water content [%w/w] of a sample.

The amount of each individual impurity (herein also referred to as related substance) may be calculated by the following formula:

$$Related\ Substances\ [\%\ w/w] = \frac{PA_{SPL}}{PA_{STD}} \times \frac{W_{STD}}{W_{SPL}} \times \frac{(P_{STD} \times CF)}{(100 - water - solvents)} \cdot 100\%$$ ,

wherien the meaning of the terms is given in the text hereinabove or hereinbelow, except that $PA_{SPL}$ refers to the peak area in the HPLC of an individual related substance. CF refers to an individual correction factor for specified and unspecified related substances in the working standards according to the following table.

| **Substance** | **CF** |
|---|---|
| DMF | 1.0 |
| Compound of Formula 3 | 2.01 |
| 4-(4-(2-hydroxyethoxy)-5-(2-methoxy phenoxy)-6-((5-methylpyridine)-2-sulf onamido)pyrimidin-2-yl)picolinamide | 1.25 |
| Impurity 2 (cf. Reference example 2) | 1.30 |
| Compound of Formula 6 | 1 |
| N-(2-(2-(1H-tetrazol-5-yl)pyridin-4-yl)-6-hydroxy-5-(2-methoxyphenoxy) pyrimidin-4-yl)-5-methylpyridine-2-sulfonamide | 0.79 |
| Compound of Formula 4 | 1.33 |
| N,N'-((ethane-1,2-diylbis(oxy))bis(2-(2-(1H-tetrazol-5-yl)pyridin-4-yl)-5-(2-methoxyphenoxy)pyrimidine-6,4-diyl)) bis(5-methylpyridine-2-sulfonamide) | 0.93 |
| Any unspecified ≥ 0.05 % w/w | 1 |

## Photometric titration

**Equipment:** Titrino (e.g. model 716 by Metrohm). **Electrode:** Combined pH-electrode (e.g. Metrohm 6.0232.100). **Titration rate:** measurement drift: 10 mV/min, max. waiting period 60 s, measuring point density: 4, min. increment: 10.0 μL, dosing rate: maximum mL/min. **Solvent** Water. **Reagent:** hydrochloric acid 0.1 mol/L. **Standard:** Trizma base. **Sample concentration:** approx.1.9 mg/mL. **Temperature:** Ambient.

## X-ray powder diffraction (XRPD)

X-ray diffractograms were measured on a Bruker D8 Advance diffractometer with FlipStick™ sample stage, Cu Kα radiation (40 kV, 40 mA), and 1D-linear LynxEye™ detector. Samples were prepared on a silicon single crystal sample holder with a cavity of 25 mm diameter and 0.5 mm depth. The powder was spread with a microscope slide to obtain a flat surface and, in the case of the X-ray diffractogram shown in Figure 1, covered with a Kapton foil to protect it from ambient humidity. Diffractograms were collected in the reflection mode with coupled θ/2θ angles in the range from 3-50° 2θ, an increment of 0.02° and an accumulation time of 0.4 s per step. The divergence slit was set to variable slit size and the antiscatter slit to maximum opening. In the case of the X-ray diffractogram shown in Figure 1, the divergence and the antiscatter slit may be set to 0.3°. The samples were continuously rotated with 30 rpm during the measurement. 2θ values of peak positions are given with an accuracy of +/- 0.2°.

## Differential scanning calorimetry 1 (DSC1)

DSC data were collected on a Mettler Toledo DSC unit (e.g. DSC823E or DSC3+). Typically, several mg of each sample were weighed in a DSC crucible and heated from 20°C to 400°C with a heating ramp of 3-4 K/min. Peak temperatures are reported for melting points.

## Differential scanning calorimetry 2 (DSC2)

DSC measurements were performed with a Mettler Toledo DSC 3+ STARe system. 1 to 5 mg of the compounds were weighed into aluminum pans (Mettler Toledo, part no. ME-51119870 and ME-51119873) under ambient conditions. The pans were closed with an aluminum cover and automatically pierced by the autosampler of the instrument prior to the insertion into the instrument. Scans were run from -20 °C to 250 °C at a rate of 10°C/min while purging the DSC oven with a constant flow of nitrogen.

## $^1$H-NMR

**Sample:** 10 mg of material are dissolved in 0.8 mL DMSO-d6. **Equipment:** 400 MHz device; Number of scans 16; Dwell time 60.200 μs; FIDRES approx. 0.25 Hz; Temperature 293K; Rotation 20 Hz; Acquisition time approx. 3.95 s.

## Karl-Fischer titration

**Sample:** 100 mg; **Equipment:** KF-Coulometer cell without diaphragm (e.g. model 756 by Metrohm); Electrode: double Pt electrode (e.g. Metrohm 6.0341.100); Generator electrode without diaphragm (e.g. Metrohm 6.0345.100). **Parameters:** I(pol): 10 μA; Temperature: ambient; Generator current: 400 mA (without diaphragm); Titration speed: Optimal (Control range: 70.0 mV, Max. rate: maximum μg/min, min rate: 15.0 μg/min). **KF reagent:** Hydranal Coulomat AD. **KF-Standard** Hydranal-Water Standard KF-Oven 140-160°C.

## Headspace gas-chromatography (GS-HS)

**Sample:** 100 mg in 1.0 mL DMSO (for methanol, ethanol, DMF, and THF) or 500 mg in 1.0 mL DMF (for ethylene glycol). **Equipment:** Agilent GC system with headspace sampler and FID detector, equipped with column DB-624, 30 m x 0.32 mm, 1.8 μm (for methanol, ethanol, and THF), DB-624, 30 m x 0.53 mm, 3 μm (for DMF), and DB-WAX, 30 m x 0.32 mm, 0.5 μm (for ethylene glycol) Flow: 2-2.5 mL/min helium.

**Gradient:** for methanol, ethanol, and THF

| Init. Temp. [°C] | Init. Time [min] | Rate [°C/min] | Final Temp. [°C] | Final Time [min] |
|---|---|---|---|---|
| 40 | 5.00 | 2<br>5<br>30 | 45<br>80<br>250 | 0<br>0<br>1.83 |

**Gradient:** for DMF

| Init. Temp. [°C] | Init. Time [min] | Rate [°C/min] | Final Temp. [°C] | Final Time [min] |
|---|---|---|---|---|
| 35 | 8.00 | 20 | 250 | 5 |

**Gradient:** for ethylene glycol

| Init. Temp. [°C] | Init. Time [min] | Rate [°C/min] | Final Temp. [°C] | Final Time [min] |
|---|---|---|---|---|
| 135 | 8.00 | 30 | 240 | 3 |

**Example 1** - **Synthesis of 5-methyl-pyridine-2-sulfonic acid 6-chloro-5-(2-methoxy-phenoxy)-2-(1-oxy-pyridin-4-yl)-pyrimidin-4-yl amide**

4-(4,6-dichloro-5-(2-methoxy-phenoxy)-pyrimidin-2-yl)-pyridine-1-oxide (CAS RN 180385-16-4) and 5-methyl-pyridine-2-sulfonamide (CAS RN 65938-77-4) are commercially available substances.

4-(4,6-dichloro-5-(2-methoxy-phenoxy)-pyrimidin-2-yl)-pyridine-1-oxide (1eq.) and $K_2CO_3$ (2 eq.) were suspended in ACN (2.77 kg/kg pyridine-1-oxide) and heated to reflux. 5-methyl-pyridine-2-sulfonamide (1.05 eq.) in ACN (6.36 kg/kg sulfonamide) was added over 15-60 min and the reaction mixture stirred further for at least 6 h until conversion > 98.0%. The resulting suspension was cooled to 70°C. Aq. HCl (1M) was added until target of pH 2-4. Water (8 kg/kg pyridine-1-oxide) was added dropwise over a period of about 20 min. The suspension was cooled to 0°C over at least 90 min, stirred for at least 60 min and filtered. After filtration the residual solid was washed with water (8 kg/kg pyridine-1-oxide) at 5°C and then with ACN (2.37 kg/kg pyridine-1-oxide) at the same temperature. After drying 5-methyl-pyridine-2-sulfonic acid 6-chloro-5-(2-methoxy-phenoxy)-2-(1-oxy-pyridin-4-yl)-pyrimidin-4-yl amide free acid (yield 90%) as a beige solid material with a melting point of 166°C (DSC1) and purity of 99% (w/w, HPLC Method A) was obtained. XRPD analysis by the method disclosed herein confirmed that the product is crystalline (see Figure 2). DSC measurement (DSC2) showed an exothermic event at about 260°C, possibly due to degradation. Several large-scale batches (55-75 kg) were successfully produced using the process described above. The end product may also be isolated as a potassium salt provided that the reaction mixture is acidified (e.g. with acetic acid) to about pH 7 as opposed to pH 2-4 as described above.

**Reference Example 1** - **Synthesis of 5-methyl-pyridine-2-sulfonic acid 6-chloro-5-(2-methoxy-phenoxy)-2-(1-oxy-pyridin-4-yl)-pyrimidin-4-yl amide**

2.4 eq. of base B (Table 1) and 4-(4,6-dichloro-5-(2-methoxy-phenoxy)-pyrimidin-2-yl)-pyridine-1-oxide (1 eq.) were suspended in 4 vol. of a solvent S (Table 1) and heated to 95°C (or reflux in case of ACN). 5-methylpyridine-2-sulfonamide (1 eq.) in a solvent (4 vol.) was added dropwise under stirring and the conversion of 4-(4,6-dichloro-5-(2-methoxy-phenoxy)-pyrimidin-2-yl)-pyridine-1-oxide was measured by HPLC (Method A). Upon a satisfactory conversion, the reaction mixture was cooled to 85°C, (7 vol.) water and thereafter acetic acid (0.6 vol.) were added until pH 6-6.5 was reached. Water (7 vol.) was further added dropwise. The suspension was cooled to 4°C and stirred for 90 min. After filtration the solid was washed with dioxane/water (ratio = 1/10, 24 vol.). After drying a brown solid of 5-methyl-pyridine-2-sulfonic acid 6-chloro-5-(2-methoxy-phenoxy)-2-(1-oxy-pyridin-4-yl)-pyrimidin-4-yl amide was obtained.

Table 1

| Experiment No. | Solvent S | Base B | Conversion compound of Formula 1 -> compound of Formula 2 | Purity of compound of Formula 2 | Remarks |
|---|---|---|---|---|---|
| | | | [%] | [%] | |
| 1 | 1,4-dioxane | $NEt_3$ | 0 (after 1 day) | n.a. | |
| 2 | 1,4-dioxane | $iPr_2NEt$ | 0 (after 1 day) | n.a. | |
| 3 | 1,4-dioxane | DBU | 0 (after 4 hours) | n.a. | decomposition of starting material |
| 4 | 1,4-dioxane | DABCO | n.a. | n.a. | reaction mixture solidifies |
| 5 | 1,4-dioxane | $CaCO_3$ | 0 (after 1 day) | n.a. | |
| 6 | 1,4-dioxane | $K_2CO_3$ | 98.4 (after 16 hours) | 86.5 | |
| 7 | ACN | $K_2CO_3$ | 99 (after 16 hours) | >96 | |
| 8 | N-methyl-2-pyrrolidone | $K_2CO_3$ | 100 (after 2 hours) | 46.9 | large amount of impurities |
| 9 | N,N-dimethylformamide | $K_2CO_3$ | 100 (after 2 hours) | 44.6 | large amount of impurities |
| Conversion/purity in the table above were typically calculated based on HPLC peak areas. | | | | | |

**Example 2** - **Synthesis of 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide**

5-Methyl-pyridine-2-sulfonic acid 6-chloro-5-(2-methoxy-phenoxy)-2-(1-oxy-pyridin-4-yl)-pyrimidin-4-yl amide (1 eq.) and BHT (0.5 eq.) were suspended in ethylene glycol (6.66 kg/kg pyridine-N-oxide) and toluene (3.48 kg/kg pyridine-N-oxide) and heated to 103-107°C. After initial distillation of about 3.0 vol. of solvent at normal pressure (removal of residual water), a hot (80-85°C) solution of NaOH (5.7 eq.) in ethylene glycol (5.55 kg/kg pyridine-N-oxide) was added dropwise over at least 20 min and the reaction mixture was stirred for 90-120 min until conversion is ≥ 98.0%. The suspension was cooled to 70-75°C and the toluene was removed by distillation under reduced pressure. The temperature of the reaction mixture was set to 50-60°C and methanol (3.16 kg/kg pyridine-N-oxide) was added over at least 10 min, followed by cooling the mixture to 0-5°C over at least 60 min and further stirring for at least 60 min. The resulting suspension was filtered, and the solid residue washed with THF (2 times 1.78 kg/kg pyridine-N-oxide) and with MeOH (2.37 kg/kg pyridine-N-oxide) at 0-5°C. The mother liquor was drained. The solid was suspended in a mixture of MeOH (3.56 kg/kg pyridine-N-oxide) and water (0.5 kg/kg pyridine-N-oxide), and heated to 80-90°C for at least 60 min. After cooling to 20°C over at least 60 min the suspension was stirred further for at least 30 min. Further filtration and washing with MeOH (2.37 kg/kg pyridine-N-oxide) furnished after drying 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide sodium salt hydrate (80-85%) as off-white solid material with purity of >99% (w/w, HPLC Method A). XRPD analysis by the method disclosed herein confirmed that the product is crystalline (see Figure 3). DSC measurement (DSC2) showed an endotherm in the near 220°C, possibly attributable to melting, followed by exothermic degradation above 230°C. Several large-scale batches (44-90 kg) were successfully produced using the process described above.

**Reference Example 2** - **Synthesis of 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide**

3 g of 5-Methyl-pyridine-2-sulfonic acid 6-chloro-5-(2-methoxy-phenoxy)-2-(1-oxy-pyridin-4-yl)-pyrimidin-4-yl) was suspended in ethylene glycol (5 to 10 vol.) and heated to about 105°C. A base (see Table below) in form of a warm solution (4 to 6 eq. base in 5 vol. ethylene glycol) or in solid form was added. After stirring for a predefined amount of time (see Table 2a), the amount of converted starting material was measured by HPLC Method A described herein. In the cases in which the reaction was not discarded, the solution was cooled to 90°C and aq. HCl was added until pH = 3. Further, water was added dropwise and the suspension was cooled to 20°C, and filtered. Wash up with water and MeOH afforded 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide as off-white solid. If acetic acid was used to neutralize the reaction mixture until pH of about 5.5 is reached, the sodium salt hydrate of 6-(2-hydroxy-ethoxy)-5-(2-methoxy-phenoxy)-2-(1-oxy-pyridin-4-yl)-pyrimidin-4-yl amide was isolated.

The meaning of the term "base" in the above process is given in Table 2a/b/c.

**Table 2a**

| Exp. | Base | Conversion compound of Formula 2 -> compound of Formula 3 after 2 hours | Yield of compound of Formula 3 | Impurity 1 | Impurity 2 | Impurity 3 | Remarks |
|---|---|---|---|---|---|---|---|
| | [molar equivalents] | [%] | [%] | [%, ala] | [%, ala] | [%, ala] | |
| 1 | $K_2CO_3$ (4 eq.) | 60 | n.a. | n.a. | n.a. | n.a. | low conversion |
| 2 | KOtBu (4 eq.) | 93 | n.a. | n.a. | n.a. | n.a. | insufficient conversion |
| 3 | $Cs_2CO_3$ (4 eq.) | 33 | n.a. | n.a. | n.a. | n.a. | low conversion |
| 4 | NaH (4 eq.) | 96 | n.a. | n.a. | n.a. | n.a. | very exothermic reaction |
| 5 | KOH (5 eq.) | 98.4 | 85.6 | 8.2 | 0.2 | 1.1 | impurity 1 too high |
| 6 | NaOH (5 eq.) | 94.3 | 80.2 | 0.5 | 0.1 | 0.4 | conversion slow, but impurity 1 reduced |

**Table 2b**

| Exp. | Base | Time when highest purity is observed | Conversion compound of Formula 2 -> compound of Formula 3 | Purity Formula 3 | Impurity 2 | Remarks |
|---|---|---|---|---|---|---|
| | [molar equivalents] | | [%] | [%, ala] | [%, ala] | |
| 7 | NaOH (6 eq.) | 150 min | 95.6 | 91.7 | 1.2 | no additive |
| 8 | | 150 min | 96.9 | 93.6 | 0.3 | BHT (0.1 eq.), 1 vol. Toluene |
| 9 | | 180 min | 99.6 | 95.4 | 0.2 | BHT (0.5 eq.), 1 vol. Toluene |

**Table 2c**

| **Exp.** | **Purging of residual compound of Formula 2 during work-up** | **Purging of Impurity 1 during work-up** | **Purging of Impurity 2 during work-up** | **Purging of Impurity 3 during work-up** | **Isolation** |
|---|---|---|---|---|---|
| | [%] | [%] | [%] | [%] | |
| 10 | -1 | +17 | -17 | +22 | Isolation of **free acid,** (mean of 8 experiments) |
| 11 | -45 | -85 | -32 | +4 | Isolation of **sodium salt,** (mean of 16 experiments) |
| The negative values in Table 2c refer to a reduction of the corresponding impurity; the positive - to enrichment thereof. | | | | | |

The impurities mentioned in Tables 2a/b/c have the following structures:

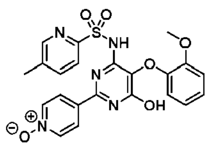

Impurity 1

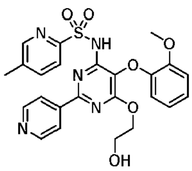

Impurity 2

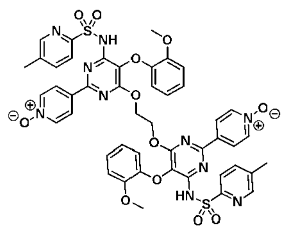

Impurity 3

The reaction of 5-Methyl-pyridine-2-sulfonic acid 6-chloro-5-(2-methoxy-phenoxy)-2-(1-oxy-pyridin-4-yl)-pyrimidin-4-yl), ethylene glycol and sodium metal as disclosed in EP0897914 is not desirable due to potential safety issues (e.g. explosion) in large-scale manufacturing. In addition, the use of sodium metal may lead to increased levels of by-products (see e.g. Niphade, et al.:Org. Process Res. Dev. 2011, 15, 1382-1387). Reduction of pyridine-N-oxides in the presence of a base and alcohols is discussed by Bjørsvik et al.: J. Org. Chem. 2005 70 (8), 3218-3224, DOI: 10.1021/jo047919b.

**Example 3** - **Synthesis of 5-methyl-pyridine-2-sulfonic acid 2-(2-cyano-pyridin-4-yl)-6-(2-hydroxy-ethoxy)-5-(2-methoxy-phenoxy)-pyrimidin-4-yl amide**

5-methyl-pyridine-2-sulfonic acid 6-(2-hydroxy-ethoxy)-5-(2-methoxy-phenoxy)-2-(1-oxy-pyridin-4-yl)-pyrimidin-4-yl amide sodium salt (1 eq.) was suspended in ACN (4.71 kg/kg pyridine-N-oxide). 2.4 vol. of the ACN were then distilled off at normal pressure (azeotropic distillation for water removal) and further ACN (1.90 kg/kg pyridine-N-oxide) was added. The reaction mixture was then cooled to 20-25°C. $NEt_3$ (1.4 eq. per "acidic" H) was added and the mixture was cooled to 0-5°C. TMS-Cl (1.15 eq. per alcohol moiety) was added over a period of at least 20 min. Directly afterwards TMS-CN (4.85 eq. per pyridine-N-oxide) was added over at least 20 min at 0-10°C. The reactor was closed, and the mixture heated to 90-95°C, pressurized with nitrogen (6-7 bar) and stirred at this temperature for at least 16 hours. The reaction mixture was then brought to 55-60°C and acetic acid (1.96 kg/kg pyridine-N-oxide) was dosed over at least 30 min. Water (2 kg/kg pyridine-N-oxide) was added over at least 15 min. After stirring for 30 min at 60°C 5.4 vol. of the solvent was distilled off. Water (8 kg/kg pyridine-N-oxide) was added over a period of at least 30 min. The resulting suspension was cooled to 20°C during at least 60 min, stirred for additional at least 60 min at this temperature, and filtered. The solid was washed as follows: first with ACN (1.1 kg/kg pyridine-N-oxide) / water (0.86 kg/kg pyridine-N-oxide), then with water (2 kg/kg pyridine-N-oxide), and finally twice with ACN (2 kg/kg pyridine-N-oxide). Drying furnished 5-methyl-pyridine-2-sulfonic acid 2-(2-cyano-pyridin-4-yl)-6-(2-hydroxy-ethoxy)-5-(2-methoxy-phenoxy)-pyrimidin-4-yl amide (86-96%) as a beige solid material with a purity of >98.5% w/w. XRPD analysis by the method disclosed herein confirmed that the product is crystalline (see Figure 4). DSC measurement (DSC2) showed an endothermic event at 210±2°C. Several large-scale batches (e.g. 27 kg) were successfully produced using the process described above.

**Reference Example 3** - **Synthesis of 5-methyl-pyridine-2-sulfonic acid 2-(2-cyano-pyridin-4-yl)-6-(2-hydroxy-ethoxy)-5-(2-methoxy-phenoxy)-pyrimidin-4-yl amide**

**a) Absence of TMS-Cl**

In analogy to Example 3, 5 g of 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide sodium salt was reacted with TMS-CN (3.0 eq.) in the presence of triethylamine (2.0 eq.) in ACN at reflux. After 16 hours no product was formed. Additional triethylamine (1.0 eq.) and TMS-CN (1.0 eq.) were added and the reaction carried on for 39 hours. HPLC analysis of the reaction mixture showed that about 14% of 5-methyl-pyridine-2-sulfonic acid 2-(2-cyano-pyridin-4-yl)-6-(2-hydroxy-ethoxy)-5-(2-methoxy-phenoxy)-pyrimidin-4-yl amide was formed and 85% of the starting material was still present in the reaction mixture.

**b) Absence of TMS-Cl and presence of dimethyl carbamoyl chloride**

In analogy to Example 3, 5 g of 5-methyl-pyridine-2-sulfonic acid 6-(2-hydroxy-ethoxy)-5-(2-methoxy-phenoxy)-2-(1-oxy-pyridin-4-yl)-pyrimidin-4-yl amide sodium salt was reacted with TMS-CN (2.0 eq.) and dimethyl carbamoyl chloride (1.5 eq.) in ACN (6 vol.) at r.t. After 48 hours, no conversion of the starting material was observed.

**c) Elevated reaction pressure**

It was observed that elevated pressure (e.g. 6-7 bar) in the process of Example 3 led to significantly shorter reaction times, thereby preventing the formation of impurities such as Impurity 2 (structure shown in Reference Example 2).

**Example 4** - **Synthesis of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide adsorbed on kieselguhr**

5-methyl-pyridine-2-sulfonic acid 2-(2-cyano-pyridin-4-yl)-6-(2-hydroxy-ethoxy)-5-(2-methoxy-phenoxy)-pyrimidin-4-yl amide (1 eq.) and $NH_4Cl$ (2.48 eq.) were suspended in DMF (4.06 kg/kg per cyano-pyridine). A solution of $NaN_3$ (2.48 eq.) in water (0.9 kg/kg cyano-pyridine) was added at 20-25°C within 15 min. The mixture was heated to 95°C over at least 60 min, stirred at this temperature for 12-15 h, and cooled down. At 20-25°C a solution of $NaNO_2$ (5.04 eq.) in $H_2O$ (1.8 kg/kg cyano-pyridine) was added over at least 40 min. The resulting reaction mixture was heated up to 40-45°C over at least 20 min and dosed with aq. HCl (2M, approx. 3.9 kg/kg cyano-pyridine) over at least 60 min until pH of about 3.3 was reached. The suspension was heated to 30°C and filtered over filter aid Hyflo Super-Cel NF™ (1.00 kg/kg cyano-pyridine; pre-washed with aqueous HCl and water). After filtration at 45°C the solid was slurry-washed at the same temperature with $H_2O$ (3.30 kg/kg cyano-pyridine) and then filtered. The solid residue was further slurry-washed with MeOH (3.86 kg/kg cyano-pyridine) and filtered, followed by another slurry-wash with EtOH (3.86 kg/kg cyano-pyridine) and final filtration. Drying of the solid furnished 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide adsorbed on Hyflo® Super Cel® NF (91-100%) as off-white solid. Several large-scale batches (38-48 kg with regard to the final product attached to the adsorbent) were successfully produced using the process described above.

5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide was obtained in Example 4 as very fine particles which made its direct filtration nearly impossible in large-scale manufacturing. The addition of a filter aid such as Hyflo® Super Cel® NF allowed for a significant improvement in filtration rate. It was observed that when the reaction mixture was filtered at 20°C over a pad of 100% w/w Hyflo® Super Cel® NF the filtration took about 13 min on a 5 g-scale. Reducing the amount of filtration aid by 50%, doubled the filtration time. Thus, filtration without filter aid or with a reduced amount thereof was not considered viable for large-scale manufacturing.

**Example 5** - **Synthesis of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide or a tetrahydrofuran solvate thereof**

5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide adsorbed on Hyflo Super-Cel NF™ as obtained in Example 4 was suspended in THF (47.6 kg/kg cyano-pyridine) at reflux, stirred over at least 90 min at this temperature and filtered. The solid residue was again suspended in THF (about 6 kg/kg cyano-pyridine) at reflux, stirred over at least 20 min at this temperature and filtered. The combined filtrates were concentrated by distillation of THF (53.5 vol.) at normal pressure. The product solution was cooled to 10-15°C over at least 60 min, stirred for at least 60 min and filtered. Slurry-washing the solid residue with EtOH (3 kg/kg cyano-pyridine), then with MeOH (3 kg/kg cyano-pyridine); and drying at ≤ 80°C under vacuum for at least 2 hours furnished 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide as solid material. XRPD analysis by the method disclosed herein confirmed that

the product is crystalline (see Figure 5). DSC measurement (DSC2) showed an endotherm at about 245-250°C, followed by exothermic decomposition. Optionally, warm (61-63°C) THF (about 5 kg/kg cyano-pyridine) was added to the solid material and the resulting suspension was stirred at reflux for at least 60 min. The product suspension was cooled to 20°C over at least 60 min, further stirred for at least 30 min, filtered, and the filter cake slurry washed with THF at 20-25°C. After disposal of the mother liquor and the wash solution, the filter cake was pre-dried at ≤ 80°C and further dried at the same temperature under vacuum to furnish 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide tetrahydrofuran solvate (75-90%) as a white solid material. XRPD analysis by the method disclosed herein confirmed that the product is crystalline (see Figure 6). DSC measurement (DSC2) showed a broad endotherm at about 160°C attributable to loss of THF, and an endotherm at about 245°C, followed by exothermic decomposition. Several large-scale batches (14-19 kg with regard to the THF solvate) were successfully produced using the process described above.

Examples 4 and 5 as disclosed above can advantageously be combined in one single step (telescoped procedure), wherein the product adsorbed on Hyflo® Super Cel® NF is not isolated but it is directly treated with THF under the conditions disclosed in Example 5, furnishing 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide or a tetrahydrofuran solvate thereof.

**Example 6** - **Synthesis of 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide disodium salt (clazosentan disodium salt)**

A) Salt formation: 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide or a tetrahydrofuran solvate thereof (crude material; 1 eq. corr. by HPLC-assay) was suspended in MeOH (4.34 kg/kg crude) and stirred at 25 - 30°C for at least 1 h. 30% NaOMe solution in MeOH (2.55 eq.) was added at 25 - 42°C to the suspension. The reaction mixture was heated to 35 - 42°C and stirred at this temperature for 60 - 120 min. The suspension was cooled down to 20 - 25°C within 60 - 180 min and stirred at this temperature for 240 - 360 min. The suspension was further cooled to about -13°C within 120 - 240 min and further stirred for at least 180 min. The product was filtered off and the filter cake washed with cold (-15°C) MeOH (1kg/kg crude) and then with cold (-15°C) EtOH (1kg/kg crude). The raw product was dried in vacuo at T ≤ 70°C resulting in solid 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide disodium salt.

B) Recrystallization: 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide disodium salt as obtained from step A) was suspended in water (2.8 kg/kg crude), the resulting suspension heated to 75-80°C over at least 30 min and maintained at the this temperature for at least 10 min, followed by addition of hot aqueous solution of NaOH (20%, 0.3 eq.) at the same temperature under stirring until a clear solution was obtained (8-12 min). Upon filtration through a pre-heated filter the solution was cooled to about 40 °C over a period of 60 to 120 min and further stirred for 90-180 min until crystallization started. The formed suspension was then cooled to 0 to 5 °C over a period of 2-24 h and stirred for 1-24 h. The mother liquor was drained off. The remaining solid was stirred for at least 10 min at 2°C in MeOH (1 kg/kg crude) followed by drainage of the solvent and further washing for at least 10 min at 2°C in EtOH (1 kg/kg crude). Finally, the mother liquor was drained off and a solid product was obtained.

C) Trituration (reslurry): The solid material from step B) was suspended in hot (70°C) MeOH / EtOH mixture (2 kg/kg crude / 6 kg/kg crude), heated to reflux (90 - 95°C) over at least 20 min and stirred for at least 60 min. The solvent was partially distilled (1.5 vol.) at atmospheric pressure. The remaining suspension was cooled to -15 °C over a period of at least 180 min, stirred for at least 120 min, and the solid material was separated from the mother liquor. The solid product was further washed in MeOH / EtOH mixture (0.3 kg/kg crude / 0.8 kg/kg crude) for at least 20 min at -15 °C, and then washed again in EtOH (1 kg/kg crude) for at least 20 min at -15 °C. Finally, the mother liquor was drained off and a solid material was obtained.

D) Drying: The solid material from step C) was pre-dried at ≤ 70°C under reduced pressure until no condensate was observed, and further dried at ≤ 75°C under vacuum to furnish 5-methyl-pyridine-2-sulfonic acid {6-(2-hydroxy-ethoxy)-5-(2-methoxy-phenoxy)-2-[2-(1H-tetrazol-5-yl)-pyridin-4-yl]-pyrimidin-4-yl}-amide disodium salt (clazosentan disodium salt) as a white to pale yellow crystalline powder. Several large-scale batches (13-20 kg output amount) were successfully produced using the process described above. Analytical data are summarized in Table 3.

**Table 3**

| **Measurement** | **Result** |
|---|---|
| XRPD (acc. to the method described herein) | crystalline form A; Figure 1 |
| Color (determined acc. to Ph. Eur.2.2.2.) | $GY_6$, $GY_7$ |
| Sodium content (photometric titration acc. to USP <541> / Ph. Eur. 2.2.20) | 7.3% w/w |
| Assay (HPLC method B) | 99.1% w/w |

**Reference Example 4 - Discoloration experiments**

A manufacturing process without purification step (f) resulted in clazosentan disodium salt exhibiting a significant coloration when dissolved in water. Several attempts were made to discolor the product: e.g., 1) the reaction temperature of step (c) was lowered as far as feasible for a large-scale manufacturing; 2) purity of 5-methyl-2-pyridine-sulfonamide in step (a) of the synthesis was increased from 95% w/w to 99% w/w; 3) sodium methoxide used in the salt formation step (e) was replaced by sodium hydroxide or sodium hydride as well as different amounts of sodium methoxide, sodium hydroxide, and sodium hydride were tested; and 4) adsorbent materials such as charcoal, silica gel, and aluminum oxide were added to the reaction mixture after completion of step (d). However, none of the above measures led to a satisfactory improvement in the undesired coloration of the drug substance. The issue could only be solved by introducing purification step (f). The results of the discoloration experiments are shown in Table 4 below.

Table 4

| **No.** | **Discoloration measures** | **Color** | **Sodium content, % w/w** |
|---|---|---|---|
| 1 | 1 Salt formation step (e) without purification step (f) | $GY_1$ | n.a. |
| 2 | Low temperature of step (c) | | n.a. |
| 3 | Increase of the purity of 5-methyl-2-pyridine-sulfonamide | $GY_1$ | n.a. |
| 4 | Replacement of sodium methoxide by sodium hydroxide or sodium hydride in step (e) | $GY_1$ | n.a. |
| 5 | Adsorbents added to reaction mixture in step (d) | $GY_1$, $GY_2$ | n.a. |
| 6 | Trituration in ethanol | $GY_3$ | 6.78 |
| 7 | Recrystallization in water (pH = 7.5 - 8.3) | $GY_4$ | n.a. |
| 8 | Recrystallization in water, followed by trituration in ethanol | $GY_5$ | 6.38 |
| 9 | Recrystallization in water/NaOH (pH = 11 - 14), followed by trituration in a mixture of ethanol and methanol | $GY_6$, $GY_7$ | 7.26 |

**Claims**

**1.** A compound of Formula 6

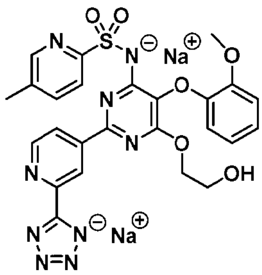

Formula 6,

**characterized in that**

• said compound has a sodium content from 7.2% w/w to 7.7% w/w;
and/or
• a test solution of 2.5% w/v of said compound in water is equally or less colored than any one of reference solutions $Y_5$, $BY_5$, $GY_5$, or $B_5$; as determined according to Chapter 2.2.2. of the European Pharmacopoeia 6.0;
and/or
• said compound is crystalline;
and/or
said compound is in a non-hydrated form;
and/or
• said compound is in a non-solvated form;
and/or
• said compound has an assay of at least 98% w/w, as determined by high-performance liquid chromatography;
and/or
• said compound comprises a total amount of impurities of not more than 2% w/w, as determined by high-performance liquid chromatography;
and/or
• said compound comprises a total amount of residual solvents of less than 1% w/w, wherein the solvents are selected from a group consisting of ethanol, methanol, tetrahydrofuran, dimethylformamide, and ethylene glycol; as determined by gas chromatography.

**2.** A compound according to claim 1, wherein said compound is crystalline, wherein said crystalline compound is **characterized by** an X-ray powder diffractogram with at least four peaks having an angle of refraction 2θ selected from: 9.4°, 12.0°, 13.2°, 17.7°, 18.4°, 19.8°, 21.2°, 21.9°, and 24.9°.

**3.** A compound according to claim 1, wherein said compound is crystalline, wherein said crystalline compound is **characterized by** the presence of peaks in the X-ray powder diffractogram at the following angles of refraction 2θ: 9.4°, 12.0°, and 21.9°.

**4.** A compound according to claim 1, wherein said compound is crystalline, wherein said crystalline compound is **characterized by** the presence of peaks in the X-ray powder diffractogram at the following angles of refraction 2θ: 9.4°, 12.0°, 18.4°, 21.2°, and 21.9°.

**5.** A compound according to claim 1, wherein said compound is crystalline, wherein said crystalline compound is **characterized by** the presence of peaks in the X-ray powder diffractogram at the following angles of refraction 2θ: 9.4°, 12.0°, 13.2°, 17.7°, 18.4°, 19.8°, 21.2°, 21.9°, and 24.9°.

**6.** A compound according to claim 1, wherein said compound is crystalline, wherein said crystalline compound is **characterized in that** the X-ray powder diffractogram essentially shows the pattern as depicted in Figure 1.

**7.** A process for the preparation of the compound of Formula 6 according to any one of claims 1 to 6, said process comprising the following steps

(e) reacting the compound of Formula 5 or a solvate thereof

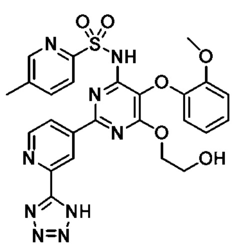

Formula 5

with a sodium containing base, to give the compound of Formula 6

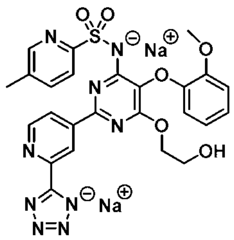

Formula 6;

(f) purifying the compound of Formula 6 by

(f1) recrystallizing the compound of Formula 6 in water at pH ≥ 7;
and
(f2) triturating the compound of Formula 6 in a solvent selected from a methanol, ethanol, or a mixture thereof;

(g) drying the compound of Formula 6.

**8.** A process according to claim 7, for the preparation of the compound of Formula 6 according to any one of claims 1 to 6, said process comprising the following steps

(a) reacting the compound of Formula 1

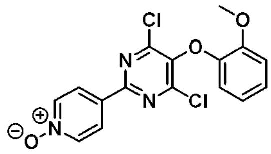

Formula 1

with 5-methyl-pyridine-2-sulfonamide or a salt thereof, under basic conditions, to give the compound of Formula 2 or a salt thereof

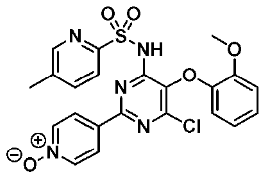

Formula 2;

(b) reacting the compound of Formula 2 or a salt thereof with ethylene glycol in the presence of an alkali metal hydroxide, to give the compound of Formula 3 or a salt thereof

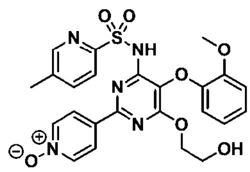

Formula 3;

(c) reacting the compound of Formula 3 or a salt thereof with trimethylsilyl cyanide in the presence of a mono-, di-, or tri-$C_{1-4}$-alkyl amine, to give the compound of Formula 4

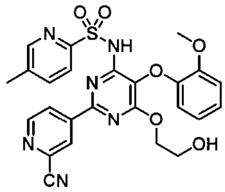

Formula 4;

(d) reacting the compound of Formula 4 with an alkali metal azide in the presence of ammonium chloride, to give the compound of Formula 5 or a solvate thereof

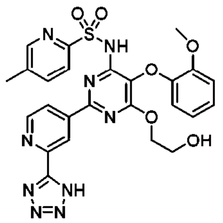

Formula 5;

and (e), (f), and (g), each according to claim 7.

**9.** A pharmaceutical composition comprising the compound according to any one of claims 1 to 6, wherein said composition further comprises at least one pharmaceutically acceptable carrier.

**10.** A pharmaceutical composition according to claim 9, said composition comprising

- from 23.5 mg/mL to 26.5 mg/mL of the compound according to any one of claims 1 to 6, wherein said concentration refers to the compound according to any one of claims 1 to 6 in its free acid form;
- from 8.0 mg/mL to 12.0 mg/mL tris(hydroxymethyl)aminomethane;
- from 0.08 mg/mL to 0.12 mg/mL disodium edetate; and
- from 2.0 mg/mL to 3.0 mg/mL sodium chloride;

wherein the pH value of said composition is from 7.0 to 9.0.

**11.** A pharmaceutical composition according to claim 10, wherein said composition is equally or less colored than reference solution $GY_3$, as determined according to Chapter 2.2.2. of the European Pharmacopoeia 6.0.

**12.** The compound according to any one of claims 1 to 6, or a composition according to any one of claims 9 to 11, for use as a medicament.

**13.** The compound according to any one of claims 1 to 6, or a composition according to any one of claims 9 to 11, for use in the prevention of cerebral vasospasm, and vasospasm-related cerebral infarction and cerebral ischemic symptoms after aneurysmal subarachnoid hemorrhage surgery/securing.

**14.** The compound according to any one of claims 1 to 6, or a composition according to any one of claims 9 to 11, for use in the prevention of cerebral vasospasm, and vasospasm-related cerebral infarction and cerebral ischemic symptoms after aneurysmal subarachnoid hemorrhage surgery, wherein a pharmaceutical composition comprising the compound is administered to a patient as intravenous continuous infusion at a dosage of 10 mg/h, wherein said dosage refers to the amount of the compound in its free acid form, wherein said administration starts after surgery of aneurysmal subarachnoid hemorrhage and continues for up to 15 days after aneurysmal rupture.

**15.** The compound according to any one of claims 1 to 6, or a composition according to any one of claims 9 to 11, for use in the reduction of the incidence and/or severity of cerebral vasospasm in patients following aneurysmal subarachnoid hemorrhage.

Figure 1

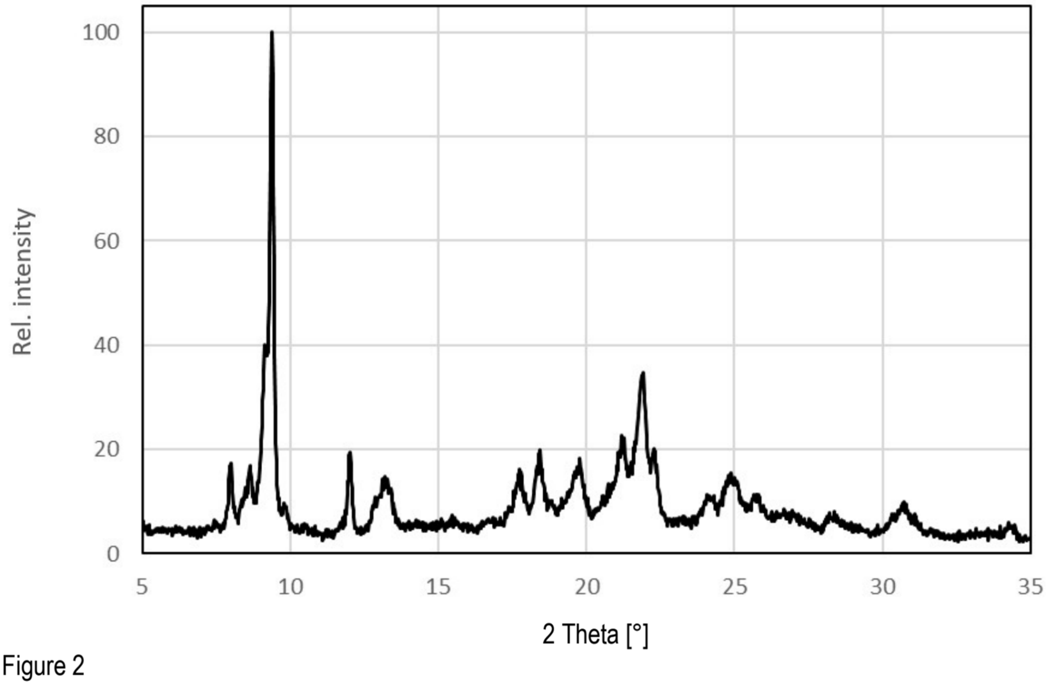


Figure 2

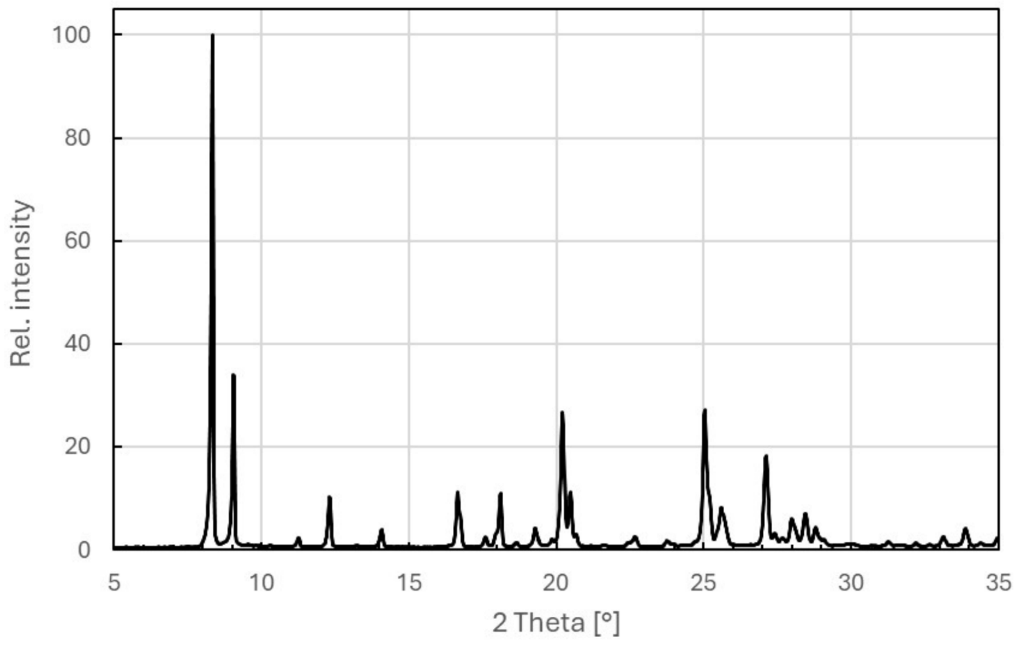

Figure 3

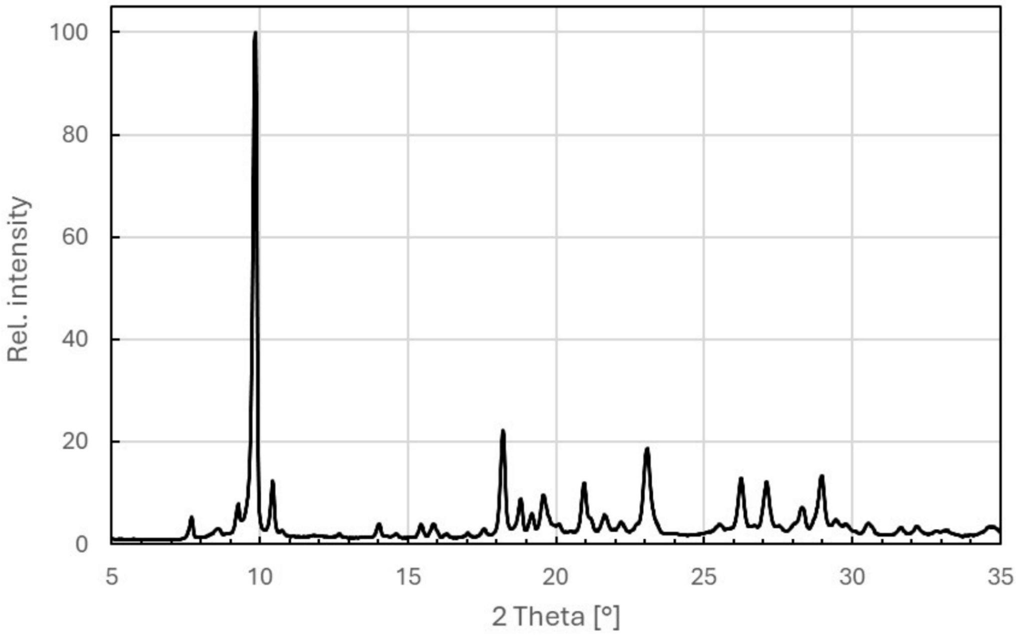


Figure 4

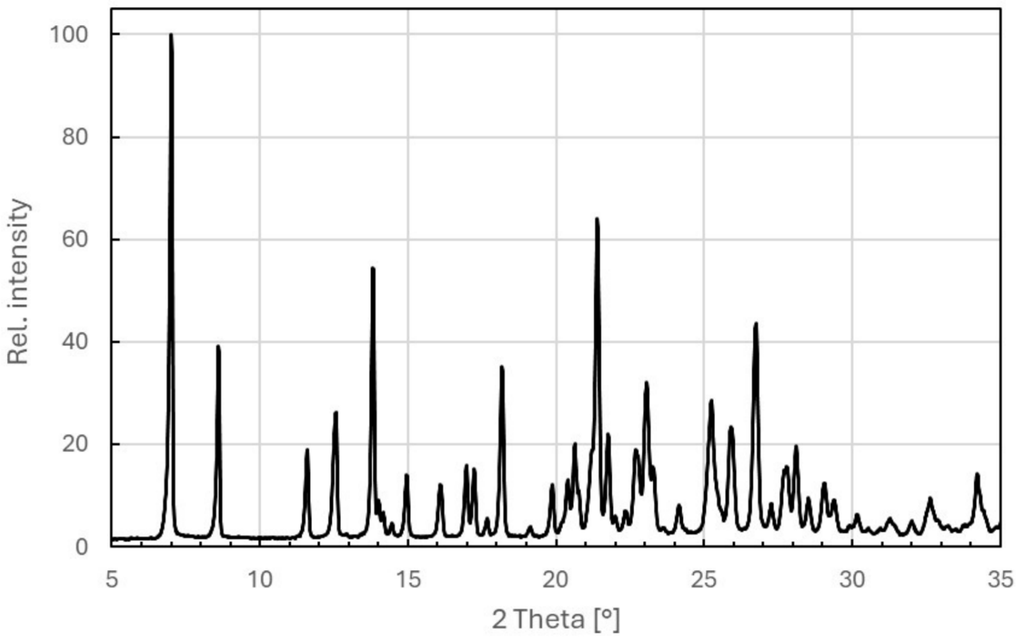

Figure 5

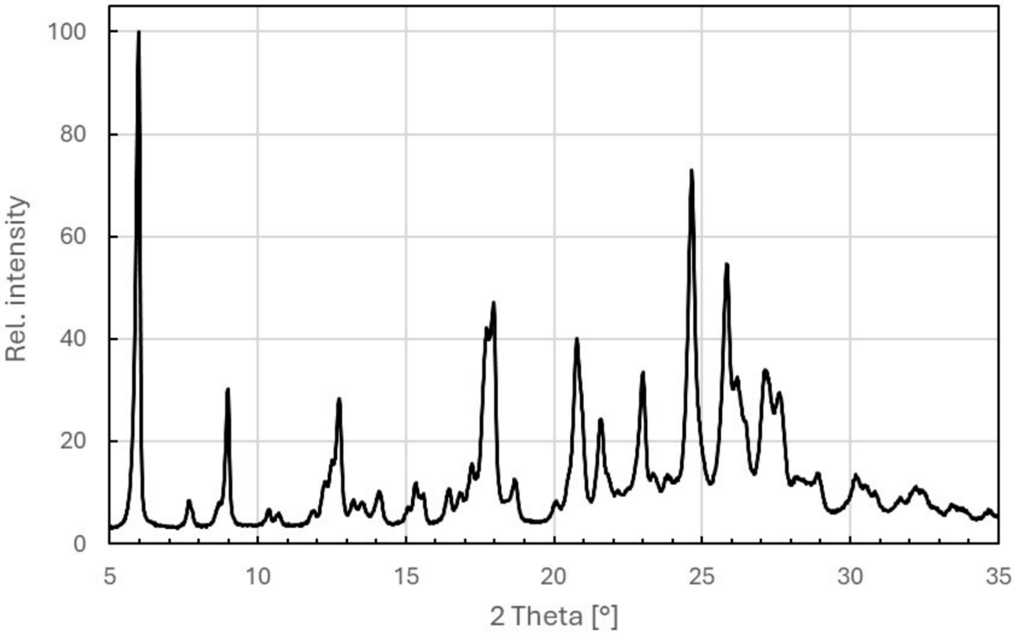


Figure 6

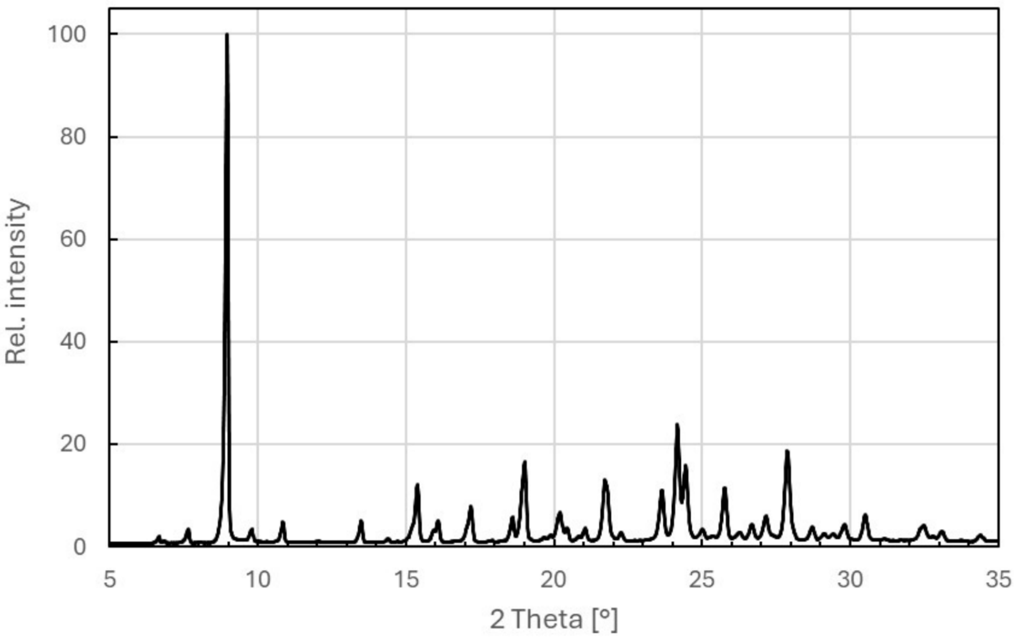

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0979822 A
- WO 9619459 A
- EP 0897914 A
- WO 2000042035 A
- WO 2000052007 A


### Non-patent literature cited in the description


- **LEE A. CLAZOSENTAN**. *First Approval. Drugs.*, April 2022, vol. 82 (6), 697-702
- **VORBRÜGGEN et al.** *Synthesis*, 1983 (4), 316-319
- **T. SAKAMOTO et al.** *Chem.Pharm.Bull.*, 1985, vol. 33, 565-571
- **A. KUBO et al.** *Chem.Pharm.Bull.*, 1988, vol. 36, 4355-4363
- Pharmaceutical Manufacturing. **REMINGTON**. The Science and Practice of Pharmacy. Lippincott Williams & Wilkins, 2005
- Polymorphism in the Pharmaceutical Industry. **U.J. GRIESSER**. The Importance of Solvates. VCH, 2006
- *CHEMICAL ABSTRACTS*, 180385-16-4
- *CHEMICAL ABSTRACTS*, 65938-77-4
- **NIPHADE et al.** *Org. Process Res. Dev.*, 2011, vol. 15, 1382-1387
- **BJØRSVIK et al.** *J. Org. Chem.*, 2005, vol. 70 (8), 3218-3224